# EUROPEAN PATENT SPECIFICATION

(11) **EP 4 665 417 B1**
(45) Date of publication and mention of the grant of the patent: **02.12.2026**
(21) Application number: 24705479.4
(22) Date of filing: 15.02.2024
(51) Int. Cl.: A61L 24/04, A61K 49/04

(54) **IN SITU GELLING LIQUID EMBOLIC FORMULATIONS AND RELATED METHODS**
IN-SITU-GELIERENDE FLÜSSIGE EMBOLIEFORMULIERUNGEN UND ZUGEHÖRIGE VERFAHREN
FORMULATIONS EMBOLIQUES LIQUIDES À GÉLIFICATION IN SITU ET PROCÉDÉS ASSOCIÉS

(30) Priority: 16.02.2023 US 202363485499 P
(43) Date of publication of application: 24.12.2025
(60) Divisional application: 26205746.6
(73) Proprietor: Biosphere Medical, S.A., 93200 Saint Denis (FR)
(72) Inventor: REB, Philippe, 95450 Themericourt (FR); THOMAS, Meriadeg, 93200 Saint Denis (FR)
(74) Representative: Cleveland Scott York
(86) International application number: PCT/EP2024/053866
(87) International publication number: WO 2024/170683

(56) References cited:
- WO-A1-2020/202210
- US-A1- 2021 277 162
- LI ET AL: "Thermosensitive N-isopropylacrylamide-N-propylacrylamide-vinyl pyrrolidone terpolymers: Synthesis, characterization and preliminary application as embolic agents", BIOMATERIALS, ELSEVIER, AMSTERDAM, NL, vol. 26, no. 34, 1 December 2005 (2005-12-01), pages 7002 - 7011, XP005000792, ISSN: 0142-9612, DOI: 10.1016/J.BIOMATERIALS.2005.05.094
- JINGJIE HU ET AL: "Advances in Biomaterials and Technologies for Vascular Embolization", ADVANCED MATERIALS, VCH PUBLISHERS, DE, vol. 31, no. 33, 6 June 2019 (2019-06-06), pages n/a, XP071874752, ISSN: 0935-9648, DOI: 10.1002/ADMA.201901071

## Description

### RELATED APPLICATIONS

This application claims priority to United States Provisional Patent Application No. 63/485,499, filed February 16, 2023, and titled IN SITU GELLING LIQUID EMBOLIC FORMULATIONS AND RELATED METHODS.

### TECHNICAL FIELD

The present disclosure relates generally to embolic formulations and methods of treatment. More specifically, the present disclosure relates to formulations comprising radiopaque monomers, polymers, and complexes thereof, and methods related thereto.

WO 2020/202210 A1 discloses a process for the preparation of a radiopaque liquid embolic composition comprising tetra iodo compound, 4,4-bis (4-hydroxy-3, 5 diiodo phenyl) valeric acid (IBHV), covalently linked to ethylene vinyl alcohol copolymer (EVOH) and dissolved in a water miscible organic liquid, wherein said organic liquid is selected from dimethyl sulfoxide (DMSO) or N-methyl pyrrolidone (NMP).

### BRIEF DESCRIPTION OF THE DRAWINGS

The embodiments disclosed herein will become more fully apparent from the following description and appended claims, taken in conjunction with the accompanying drawings. The drawings depict primarily generalized embodiments, which embodiments will be described with additional specificity and detail in connection with the drawings in which:
FIG. 1 illustrates a complex coacervation mechanism underlying an embolic solution according to an embodiment.
FIG. 2 is an NMR spectrum of raw polymerization medium after polymerization of RO acrylamide using ammonium peroxodisulfate (APS)/N,N,N',N'-tetramethylethylenediamine (TEMED) as initiator.
FIG. 3 is a comparison of FT-IR spectrograms of RO acrylamide monomer (lower trace) and poly(RO acrylamide) polymer prepared using 2,2'-Azobis[2-(2-imidazolin-2-yl)propane] dihydrochloride (VA-044) as initiator (upper trace).
FIG. 4 is a comparison of FT-IR spectrograms of a poly(RO acrylamide) polymer salt prepared using 2,2'-Azobis(2-methylpropionamidine) dihydrochloride (V-50) as initiator (lower trace) and acidic poly(RO acrylamide) polymer prepared using VA-044 as initiator (upper trace).
FIG. 5A is an NMR spectrum of poly[2-(Methacryloyloxy)ethyl] trimethylammonium chloride (polyMETAC).
FIG. 5B is a comparison of FT-IR spectrograms of the polyMETAC (upper trace) and METAC monomer (lower trace).
FIG. 6 is a comparison of FT-IR spectrograms of a copolymer of METAC and 2-hydroxyethylmethacrylate (HEMA; upper trace) and polyHEMA (lower trace).
FIG. 7 is an NMR spectrum of raw polymerzation medium from copolymerization of RO acrylamide and 2-Acrylamido-2-methylpropanesulfonic acid (AMPS).
FIG. 8 is a comparison of FT-IR spectrograms of poly(RO acrylamide-co-styrene sulfonate) (upper trace); poly(RO acrylamide) (middle trace); and poly(styrene sulfonate) (lower trace).
FIG. 9 is a set of size exclusion chromatograms of poly(RO acrylamide) polymers and copolymers in accordance with an embodiment.
FIG. 10 shows results of a static gel formation test performed on an embolic solution in accordance with an embodiment.
FIG. 11A shows a kidney vasculature model immediately prior to delivery of an embolic solution in accordance with an embodiment.
FIG. 11B shows the kidney vasculature model of FIG. 11A at commencement of delivery of the embolic solution.
FIG. 11C shows the kidney vasculature model of FIG. 11A after completion of delivery of the embolic solution.
FIG. 11D demonstrates occlusion of the kidney vasculature model of FIG. 11A by the embolic solution.
FIG. 12 shows results of a static gel formation test performed on another embolic solution in accordance with an embodiment.
FIG. 13 is a time-lapse photographic series illustrating solidification in saline of a drop of embolic solution.
FIG. 14 shows visible light and CT-scan shots of formulations containing radiopaque polymer.
FIG. 15A is a diagram of an apparatus for testing occlusion ability in embolic solutions.
FIG. 15B shows an example of an occlusion test with the apparatus shown in FIG. 15A using a colored gel.
FIG. 15C shows measurement of results of an occlusion test on an embolic solution in accordance with one embodiment.
FIG. 16 is a graphical determination of the minimum salt concentration required to quench complex coacervation for an example of one polyelectrolytes couple depending on the global polymer concentration in solution.
FIG. 17 is a graph of minimum salt concentration required to quench complex coacervation according to total polymer concentration for multiple polyelectrolyte solutions made with different batches of polyanionic polymer and a single batch of polycationic polymer.
FIG. 18 is a graph of minimum salt concentration required to quench complex coacervation according to total polymer concentration for multiple polyelectrolyte solutions made using a single batch of polyanionic polymer and different polycationic polymers.
FIG. 19 shows results of a static gel formation test performed using a model of *in vitro* static injection of a radiopaque embolic liquid in accordance with an embodiment.
FIG. 20 shows embolic material formed from the radiopaque embolic liquid used in the test shown in FIG. 19.

### DETAILED DESCRIPTION

Compositions comprising radiopaque monomers, polymers, and methods related thereto are disclosed herein. It will be readily understood that the embodiments as generally described below and as illustrated in the Examples and Figures could be modified in a wide variety of ways. Thus, the following more detailed description of various embodiments, as described below and represented in the Examples and Figures, is not intended to limit the scope of the disclosure, but is merely representative of various embodiments.

As used herein, the term "alkyl" as employed herein by itself or as part of another group refers to a saturated aliphatic hydrocarbon straight chain or branched chain group having, unless otherwise specified, 1 to 20 carbon atoms (whenever it appears herein, a numerical range such as "1 to 20" refers to each integer in the given range; *e.g*., "1 to 20 carbon atoms" means that the alkyl group can consist of 1, 2 or 3 carbon atoms, or more carbon atoms, up to a total of 20). An alkyl group can be in an unsubstituted form or substituted form with one or more substituents (generally one to three substituents can be present except in the case of halogen substituents, *e.g*., perchloro). For example, a C₁₋₆ alkyl group refers to a straight or branched aliphatic group containing 1 to 6 carbon atoms (*e.g*., including methyl, ethyl, propyl, isopropyl, butyl, *sec*-butyl, *tert*-butyl, 3-pentyl, hexyl, etc.), which can be optionally substituted.

As used herein, "amino" refers to an -NR^{x}R^{y} group, where R^{x} and R^{y} are independently selected from the group consisting of hydro, alkyl, cycloalkyl, aryl, heteroaryl, and heterocycle, each being optionally substituted.

The present disclosure encompasses radiopaque embolic liquids. Current embolic agents, which include cyanoacrylate adhesives and materials using dimethylsulfoxide (DMSO) as a solvent, can have significant drawbacks. Drawbacks of cyanoacrylate adhesives include unpredictability of flow and polymerization, a tendency to adhere to catheters, and elevated risk of local inflammation. DMSO is toxic and is also incompatible with some catheter materials.

In contrast to some other embolic materials, the embolic liquids described herein are in a ready-to-use liquid form that is easily delivered through a syringe and microcatheter at the target; aqueous and solvent-free; and compatible with medical delivery devices. In some embodiments of the radiopaque embolic liquid, the liquid may comprise two oppositely charged polyelectrolytes--a polyanionic polymer and a polycationic polymer--and a chaotropic salt in aqueous solution. The properties and concentrations of the polymers and salt in the radiopaque embolic liquid are selected to provide a radiopaque embolic liquid that is an injectable liquid. Once delivered to a location (e.g., a blood vessel) containing a flow of lower osmolality medium (e.g., blood plasma) the polyelectrolytes quickly undergo complex coacervation to precipitate as a hydrogel that can act as an embolic material. The resulting material is radiopaque i.e., visible in CT scan or radiographic imaging.

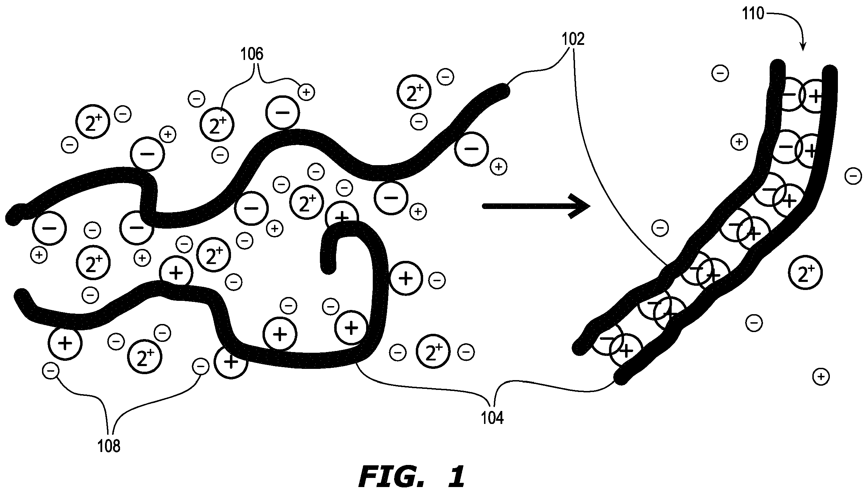

Components of such an embolic liquid are illustrated in FIG. 1. The liquid can comprise polymer salts that, upon release of their associated counterions, form oppositely charged polyelectrolytes comprising a polyanionic polymer 102 and a polycationic polymer 104, as well as their countercations and counteranions (not depicted). The liquid can further comprise cations 106 and anions 108 provided by dissociation of a chaotropic salt. As shown, the ions 106, 108 may be monovalent and/or bivalent (which can also be referred to as divalent).

Electrostatic interaction between oppositely charged polyelectrolytes results in the formation of polyelectrolyte complexes. As illustrated on the left side of the diagram, the amount of chaotropic salt present in the polyelectrolyte solution provides sufficient ionic strength that the degree of electrostatic interaction between the polyanionic polymer 102 and the polycationic polymer 104 is limited by the ions 106, 108. The resulting nonstoichiometric polyelectrolyte complexes retain some of the solvent and constitute a dense polyelectrolyte-rich fluid phase in equilibrium with the polyelectrolyte-depleted phase of remaining solvent, so that the polyelectrolyte solution remains a uniform, clear liquid. When the polyelectrolyte solution is exposed to a lower osmolality environment, as illustrated on the right side of the diagram, this thermodynamic equilibrium is disrupted by local dilution of the ions 106, 108. Increased electrostatic interaction between the polyanionic polymers 102 and the polycationic polymers 104 to form stoichiometric complexes 110 results in formation of a gel precipitate. While complex coacervation as a process can encompass formation of polyelectrolyte complexes exhibiting a range of increasing densities, the terms "coacervation" and "complex coacervation" as used herein refer particularly to the formation of a gel precipitate upon introduction into a low osmolarity environment.

In some embodiments, the polyanionic polymer comprises a backbone with a plurality of anionic monomers, i.e., monomers having groups that bear a net negative charge at or near neutral pH. The anionic groups can be integrated into the backbone or pendent therefrom. In some embodiments, one or more anionic monomers of the polyanionic polymer is a radiopaque monomer. As used herein in reference to a chemical entity, "radiopaque" describes a chemical entity comprising one or more moieties, or atoms thereof, that confer radiopacity to a polymer into which said entity is incorporated. In some embodiments, the radiopaque monomer is iodinated.

In some embodiments of radiopaque polyanionic polymers, said polymers comprise a monomer according to Formula I: 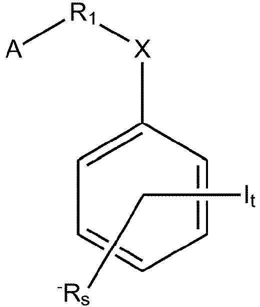 wherein R⁻ is an anionic substituent selected from COO⁻, SO₃⁻, PO₄³⁻, and PO₃²⁻; X is a linker selected from -C-, -NH-, and -CONH-; R₁ is a spacer comprising an alkyl, optionally substituted with one or more of -O-, -OH, and -CO; A is a polymerizable function; s is 1 to 3, *t* is 1 to 3, and *s* + *t* is less than or equal to 5. Examples of polymerizable functions encompassed by A include, but are not limited to, acrylamide, methacrylamide, acrylate, methacrylate, vinyl, vinylidene, and lactone.

In some embodiments, an anionic monomer according to Formulae I, Ia, Ib, and Ic can be made by grafting a linear C₂-C₁₄ aminoalkyl sequence onto an iodinated amino benzene sulfonic acid, an iodinated phenol sulfonic acid, or iodinated amino isophtalic acid, thereby giving access to a more reactive amine. For example, the aminoalkyl sequence may comprise a protected amine at one end and a good leaving group at the other for nucleophilic substitution on the -NH₂ function on the benzyl group. After removal of the protecting group, a polymerizable function such as (meth)acryloyl can then be added onto the deprotected amine with higher yields. By way of example, in some embodiments radiopaque polyanionic polymers can comprise a monomer according to Formula Ia: 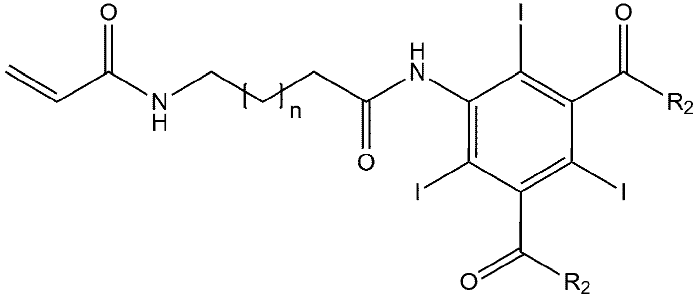 wherein n is 0-12 and wherein R₂ is either an oxygen-containing moiety or a nitrogen-containing moiety.

In another embodiment, radiopaque polyanionic polymers can comprise a monomer according to Formula Ib: 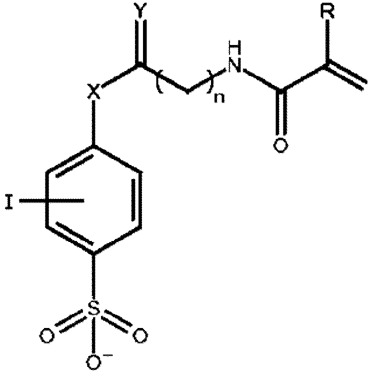 wherein R is CH₃ or H; X is O or NH; Y is O or H₂; and n is 1-4.

In another embodiment, radiopaque polyanionic polymers can comprise a monomer according to Formula Ic: 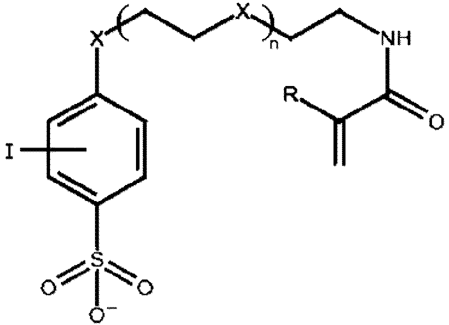 wherein R is CH₃ or H; X is O or NH; and n is 1-4.

Table 1 presents a non-exhaustive list of exemplary anionic monomers according to Formula I:

**Table 1. Examples of anionic monomers according to Formula I.**

| **Compound No.** | **Structure** | **Name** |
|---|---|---|
| Compound 1 | 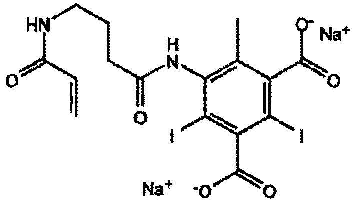 | N-amido-triiodoisophtalic acid butanamido acrylamide sodium salt ("RO acrylamide") |
| Compound 2 | 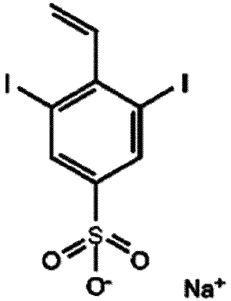 | 3,5-diiodo-4-styrenesulfonate sodium salt |
| Compound 3 | 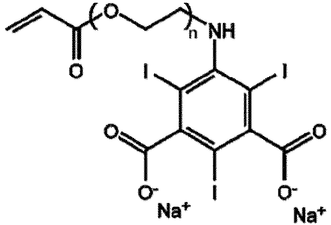 | N-amido-triiodoisophtalic acid polyethylenoxyacrylate |
| Compound 4 | 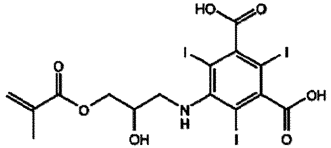 | N-amino-triiodoisophtalic acid propan-2-ol methacrylate |
| Compound 5 | 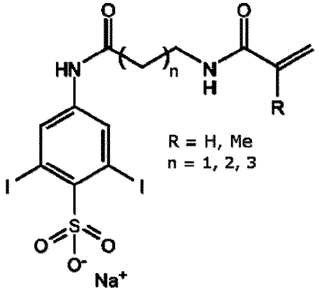 | N-amido-2,6-diiodobenzenesulfonate-alkyl-(meth)acrylamide sodium salt |
| Compound 6 | 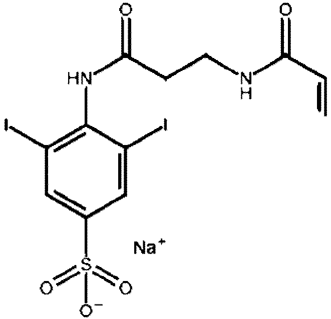 | N-amido-3,5-diiodobenzenesulfonate-ethyl-acrylamide sodium salt |

In some embodiments, conferring radiopacity onto an existing polyelectrolyte can comprise post-polymerization addition of radiopaque species directly to monomers in the polymer backbone. For example, iodine may be added by chemical reaction after polymerization to avoid addition onto the polymerizable function. Examples of anionic monomers that may be iodinated post-polymerization include, without limitation, aromatic monomer units, including polystyrene derivatives and polybenzyl acrylate derivatives, having groups such as benzoic acid, phthalic acid, benzene sulfonic acid, benzene sulfinic acid, phenylphosphonic acid, phenylphosphoric acid and phenylphosphonic acid.

In some embodiments, a radiopaque polyanionic polymer can be a copolymer of anionic monomers and radiopaque monomers for example, according to Formula II: 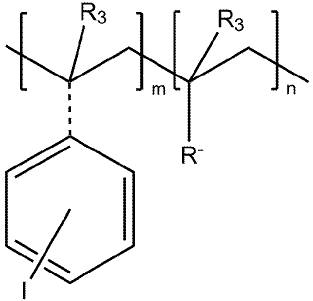 wherein R⁻ is defined as above, and R₃ is H or methyl.

In various embodiments, a radiopaque polyanionic copolymer according to Formula II can comprise a radiopaque anionic monomer according to Formula I copolymerized with a second anionic monomer such as, but not limited to, 2-acrylamido-2-methylpropanesulfonic acid (AMPS), 4-vinylbenzene sulfonate, vinylsulfonic acid, 2-carboxyethylacrylate, acrylate, 3-sulfopropyl acrylate, 2-methyl-2-propene-1-sulfonic acid, vinylphosphonic acid, vinylsulfate, acrylonitrile, methyl vinyl ether-alt-maleic acid, and 3-allyloxy-2-hydroxypropane sulfonate. Other monomers that can further undergo a simple reaction pre- or post-copolymerization (hydrolysis, saponification, easy coupling) leading to an anionic moiety may also be employed as the second anionic monomer. Examples include allyl glycidyl ether, glycidyl methacrylate, alkyl acrylates and other monomers functionalized with at least one of an amine, alcohol, and thiol bearing a sulfonate or a carboxylate, e.g., gluconates, sodium 3-mercapto-1-propanesulfonate, 4-aminobenzoic acid, 4-aminobenzene sulfonate, and 5-amino-2-naphtalenesulfonic acid.

Table 2 presents a non-exhaustive list of exemplary anionic copolymers in accordance with the foregoing description:

**Table 2. Example structures of polyanionic copolymers.**

| **Compound No.** | **Structure** | **Name** |
|---|---|---|
| Compound 7 | 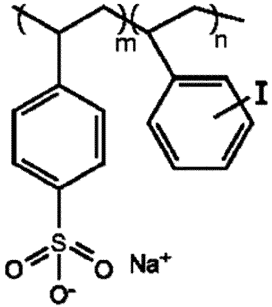 | Copolymer of styrene sulfonate sodium salt and styrene later iodinated via oxidative iodination |
| Compound 8 | 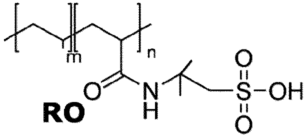 | Copolymer of N-amido-triiodoisophtalic acid butanamido acrylamide sodium salt ("RO") and AMPS |
| Compound 9 | 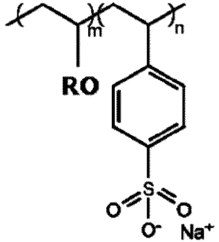 | Copolymer of N-amido-triiodoisophtalic acid butanamido acrylamide sodium salt ("RO") and styrene sulfonate sodium salt |
| Compound 10 | 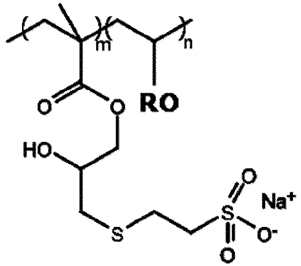 | Isethionic acid sodium salt reacted with glycidyl methacrylate and then copolymerized with N-amido-triiodoisophtalic acid butanamido acrylamide sodium salt ("RO") |
| Compound 11 | 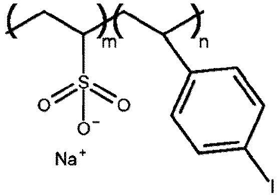 | Copolymer of vinylsulfonic acid sodium salt and 4-iodostyrene |
| Compound 12 | 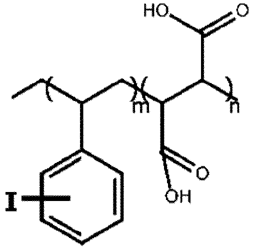 | Copolymer of iodostyrene and maleic acid obtained after copolymerization with maleic anhydride and later hydrolyzed into diacid form |

In various embodiments, the polycationic polymer is a polymerization product of one or more cationic monomers, i.e., monomers with groups that bear a net positive charge at or near neutral pH. In some embodiments, at least one of the cationic monomers of the polycationic polymer comprises one or more moieties that confer radiopacity to the polycationic polymer. In some embodiments, the radiopaque monomer is iodinated. In particular embodiments, the polycationic polymer is a polyamine compound, comprising one or more monomers that include ammonium cations, particularly quaternary ammonium cations, and/or amine groups that are charged under proper pH conditions and/or that can be quaternized by, e.g., alkylation. Other cationic monomers based on nitrogen are also contemplated, such as conjugate acids of imidazolium, pyridinium and guanidinium.

Table 3 presents a non-exhaustive list of exemplary cationic monomers in accordance with the foregoing description:

**Table 3. Example structures of cationic monomers.**

| **Compound No.** | **Structure** | **Name** |
|---|---|---|
| Compound 13 | 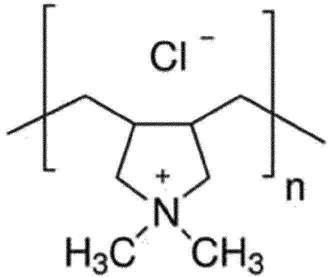 | Poly(diallyldimethyl ammonium chloride) (polyDADMAC) |
| Compound 14 | 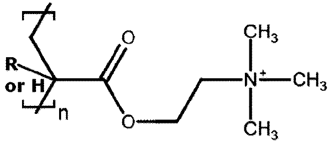 | Poly[2-(Methacryloyloxy)ethyl] trimethylammonium chloride (polyMETAC) |
| Compound 15 | 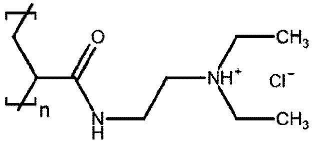 | Poly[N-(2-(Diethylamino)ethyl) acrylamide hydrochloride] (polyDEAE) |
| Compound 16 | 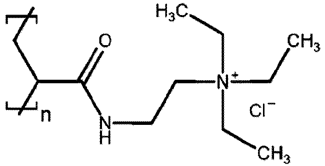 | Poly[N-(2-(triethylamino)ethyl) acrylamide chloride] - Obtained by quaternization of the diethylamino monomer (DEAE acrylamide) above |
| Compound 17 | 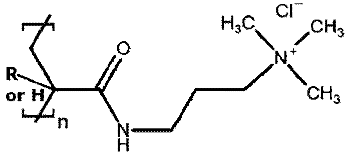 | poly[3-(Methacryloylamino)propyl]-trimethylammonium chloride |
| Compound 18 | 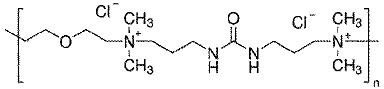 | Poly[bis(2-chloroethyl) ether-alt-1,3-bis[3-(dimethylamino)propyl]urea] (PolyQuaternium-2) |
| Compound 19 | 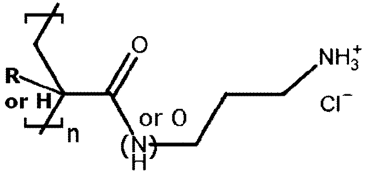 | Poly[N-(3-Aminopropyl)methacrylamide hydrochloride] |
| Compound 20 | 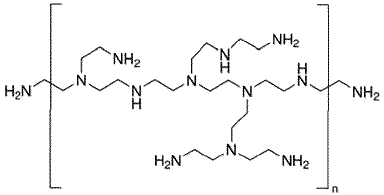 | Poly(ethyleneimine) |
| Compound 21 | 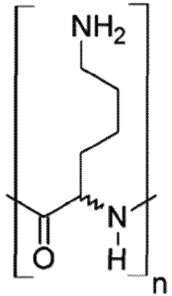 | α (alpha) polylysine |
| Compound 22 | 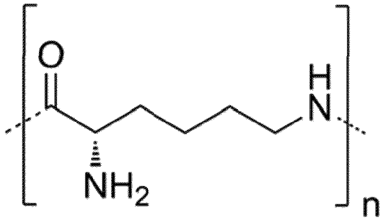 | ε(epsilon) polylysine |
| Compound 23 | 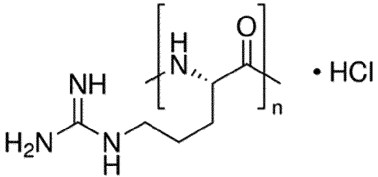 | Poly-L-arginine hydrochloride |
| Compound 24 | 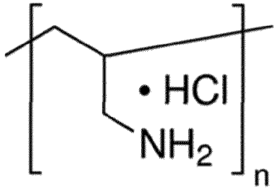 | Poly(allylamine hydrochloride) |
| Compound 25 | 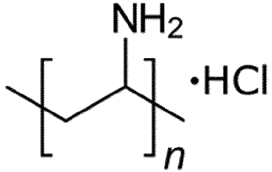 | Poly(vinylamine hydrochloride) |
| Compound 26 | 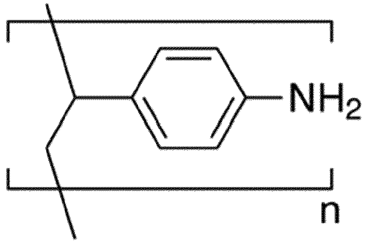 | Poly(4-aminostyrene) |
| Compound 27 | 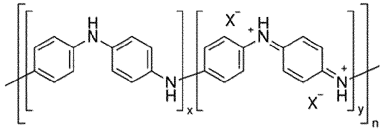 | Polyaniline (Emeraldine salt) |
| Compound 28 | 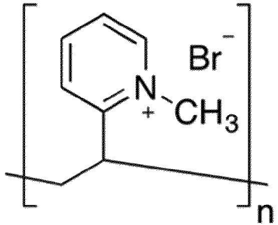 | Poly(2-vinyl-1-methylpyridinium bromide) |
| Compound 29 | 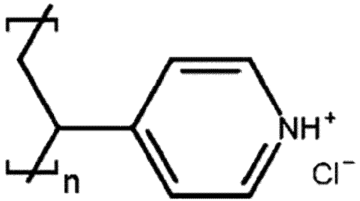 | Poly(4-vinylpyridine hydrochloride) |
| Compound 30 | 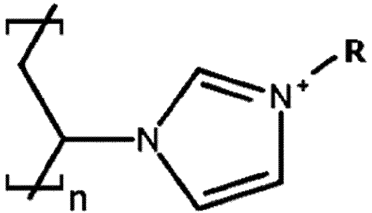 | Poly(3-vinyl-1-alkylimidazole) |
| Compound 31 | 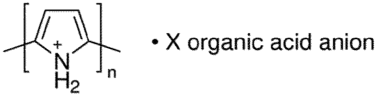 | Polypyrrole |
| Compound 32 | 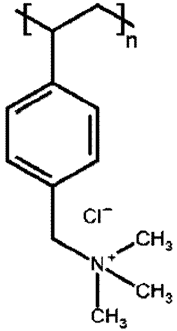 | Poly(vinylbenzyl)trimethylam monium chloride (polyVBTMAC) |

In some embodiments, a polycationic polymer can be made by coupling a quaternary ammonium to a monomer or polymer bearing reactive amine, alcohol or thiol functions, e.g., by reacting such monomer/polymer with a glycidyl ether such as glycidyltrimethylammonium chloride (GTMAC). GTMAC can be used to derivatize polysaccharides including starch, cellulose derivatives, chitosan, etc. into cationic macromolecules.

In some embodiments, compounds 26 to 32 can bear or comprise iodine substituents, such as through aromatic iodination, thus conferring radiopaque properties to the cationic polymer. The reaction may be achieved on the monomer before polymerization or directly on the polymer depending on the conditions.

In some embodiments, zwitterionic monomers or polymers are used. For example, zwitterionic monomers or polymers can be used with one or more polyanionic polymer or polycationic polymer, or can be copolymerized with one or more anionic monomer or cationic monomer. In certain embodiments, zwitterionic polymers are used in combination with one or more polyanionic polymers and one or more polycationic polymers. Zwitterionic polymers comprise cationic and anionic groups and can be employed in a wide range of applications in biomedical fields such as drug delivery and drug formulation. The following exemplary Zwitterionic polymers exhibit biocompatibility, low toxicity, and hemocompatibility.

| | | |
|---|---|---|
| Compound 33 | 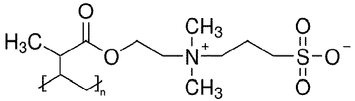 | Poly[2-(Methacryloyloxy)ethyl]dimet hyl-(3-sulfopropyl)ammonium hydroxide |
| Compound 34 | 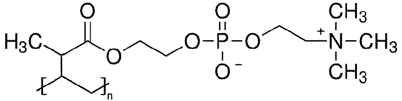 | Poly(2-Methacryloyloxyethyl phosphorylcholine) |

Charged monomers may be copolymerized with uncharged monomers in either or both of the polycationic polymer and the polyanionic polymer. In some embodiments, such uncharged monomers include acrylates and derivatives thereof such as, but not limited to acrylamide, 2-hydroxyethylmethacrylate, 2-hydroxyethylacrylate, N-isopropylacrylamide, methyl acrylate, benzyl acrylate, and N-propargyl acrylamide. This approach may be employed to affect various properties of the polyelectrolyte, for example, to space the charges in the polymeric backbone, increase mobility of the polymeric backbone, increase the molecular weight of the polymer chains, change properties such as hydrophilicity and thermosensitiveness, and to add a specific chemical function. The copolymerization rate (distribution between 2 or more different monomers in the final polymer) can then be optimized to adjust the desired properties.

The radiopaque embolic liquid includes one or more salts that provide ions to the charged functions of the polyelectrolyte molecules. The nature and concentration of the salt may be selected based upon the properties of each polyelectrolyte, the particular combination of polyelectrolytes, and the total concentration of the polyelectrolytes. In an aspect, chaotropic salts serve to quench the coacervation of the oppositely charged polymers without pH modification. In other aspects suitable salts may be described by one or more of the following: salts that are suitable for use in physiological conditions, salts that do not decompose in water, salts that are neither acidic nor alkaline, and salts that are sufficiently soluble in water to achieve an effective concentration. In another aspect, a suitable salt has a molecular weight that is sufficiently low as to provide a high molar concentration, for example up to about 5 M. Suitable salts can also have a sufficiently high relative density to lower the dilution of the final solution. For example, salts with a relative density >= 1.75 can be used.

Radiopaque embolic liquids described herein can include monovalent and/or bivalent chaotropic salts. For example, the chaotropic salt may dissociate in solution to provide cations such as Na⁺, K⁺, Mg²⁺, or Ca²⁺, and anions such as Cl⁻. In some embodiments, the chaotropic salt is a monovalent inorganic salt such as NaCl or KCl, or a bivalent inorganic salt such as CaCl₂ or MgCl₂. Other inorganic salts of similar ionic strength are contemplated, particularly salts that dissociate into ions that can be tolerated by a subject into which the radiopaque embolic liquid is introduced. In some embodiments, some organic salts can be used also, like guanidium thiocyanate It should be borne in mind that the extent to which the ions associated with the polyelectrolytes also contribute to ionic strength may depend on the dissociation of the ionic species.

Radiopaque embolic liquids according to the present disclosure comprise oppositely charged polyelectrolytes in a medium with ions of sufficient ionic strength to reduce or inhibit coacervation under *ex vivo* conditions. Upon introduction into a site to be embolized in a subject, for example a blood vessel, diffusion of the ions decreases their local concentration below a trigger concentration for coacervation of the polyelectrolytes, resulting in formation of a gel embolus comprising coacervate complexes. Accordingly, in an aspect the composition of the radiopaque embolic liquid can be configured to provide both stability of the solution before administration and rapid coacervation upon administration. As discussed below, a number of compositional features may interact to define such solutions.

The total polyelectrolyte concentration can affect the stability of the solution. For example, high concentrations may increase interaction between polyanionic polymers and polycationic polymers, creating colloidal networks and inducing precipitation. Other limiting factors on the total polyelectrolyte concentration are the solubilities of the selected polyelectrolytes in saline solution and the viscosity of the resulting solution. Solubility and viscosity are also related to the molecular weight of the selected polyelectrolytes.

As noted above, coacervation involves electrostatic interaction between opposing charges on polyelectrolytes. Accordingly, the relative proportions of the polyanionic polymer and the polycationic polymer can be selected to provide a particular charge ratio. The charge magnitude and density of each polyelectrolyte are in part a function of its monomer composition, monomer repeating pattern, and molecular weight. As also noted above, the dissociation constant of each polyelectrolyte is also a factor in that this influences the degree of ionization of the polymer in solution. In some embodiments, the relative proportions of the polyanionic polymer and the polycationic polymer can be based upon a selected stoichiometric molar ratio. This ratio may be theorized according to full dissociation of the ions in solution at a particular pH, e.g., neutral or physiological pH. In other embodiments, the relative proportions of the polyanionic polymer and the polycationic polymer can be a non-stoichiometric system that provides good stability in conjunction with a particular salt or salt mixture.

In some embodiments, the radiopaque embolic liquid comprises one of the polyanionic polymer and the polycationic polymer in excess relative to the other. Without being bound to a particular theory, in some polyelectrolyte systems the excess component may form a stabilizing shell for nanoparticles of polyelectrolyte complex in saline solution. In some polyelectrolyte systems, one polyelectrolyte may include multivalent ionic groups with the potential to bind two oppositely charged groups from two different polymer chains, resulting in cross-links that may reinforce the coacervate. In some embodiments, the polycationic polymer is present in excess. In some embodiments, the molar ratio of polycationic polymer to polyanionic polymer is about 1.05:1 to about 2.5:1 when homopolymers are used and up to about 10:1 when copolymers are involved. One consideration for such an approach may be to maintain a global positive charge for better adhesion to vascular endothelial cells, which are known to be mainly anionic.

As indicated above, the concentration of the chaotropic salt can be selected to provide sufficient ionic strength to quench coacervation of the polyelectrolytes. More particularly, the ionic strength is sufficient to stabilize the polyelectrolyte mix in a clear and fluid solution at atmospheric temperatures. While this parameter can depend on the respective properties of the polyelectrolyte couple and the salt, as a general principle the solutions described herein are highly saline. However, another consideration is the effect of salt concentration on the hardening speed of the coacervate upon introduction into a lower osmolar medium, which is dependent on the speed at which local concentration of ions drops below the trigger concentration due to diffusion. Accordingly, the concentration of the chaotropic salt can be selected with a view to both solution stability and hardening speed. In some embodiments, the salt concentration is about 0.3 M to about 6 M, or about 0.5 M to about 5 M.

The average molecular weight of each of the polycationic polymer and the polyanionic polymer can be a factor in each of the considerations discussed above. Additionally, polyelectrolytes with higher molecular weights can form gels having increased strength. However, molecular weight also affects the viscosity of the solution. Accordingly, the average molecular weight of the polyelectrolytes can be selected with a view to balancing solution fluidity and embolus strength.

Methods of making a polyelectrolyte according to the present disclosure can comprise polymerizing one or more monomers in combinations as described above. In particular, polymerization can take place in an aqueous environment with the use of one or more initiators. In some embodiments, the polymer product can be recovered and purified by e.g., dialysis or precipitation.

Methods of preparing a radiopaque embolic liquid can comprise mixing of oppositely charged polyelectrolytes in a medium with sufficient ionic strength to reduce or inhibit complexation. More particularly, a method of making a radiopaque embolic liquid can comprise combining an amount of polyanionic polymer, an amount of polycationic polymer and an amount of a chaotropic salt in aqueous solution. At least one of the polyanionic polymer and the polycationic polymer can comprise a radiopaque monomer. The amount of chaotropic salt can be selected to provide in said solution a concentration that exceeds a trigger concentration required to avoid premature complexation of the polyelectrolytes.

The polyanionic polymer can comprise a radiopaque monomer. In some embodiments, the radiopaque monomer is iodinated. In some embodiments, the polyanionic polymer comprises a radiopaque monomer according to Formulae I, Ia, Ib, or Ic. In some embodiments, the polyanionic polymer is a copolymer of monomers that are anionic and/or radiopaque, for example a copolymer according to Formula II. In some embodiments, the copolymer also includes uncharged monomers.

In various embodiments, the polycationic polymer is a polymerization product of one or more cationic monomers. In particular embodiments, the polycationic polymer is a polyamine compound comprising one or more monomers that include ammonium cations and/or amine groups that are charged under proper pH conditions and/or that can be quaternized by, e.g., alkylation. Other cationic monomers based on nitrogen are contemplated, such as conjugate acids of imidazolium, pyridinium and guanidinium. In some embodiments, the polycationic polymer can comprise a radiopaque monomer.

Combining the components of the radiopaque embolic liquid can comprise adding the components in various sequences. In various embodiments, this can involve adding the chaotropic salt to the product solution before, or simultaneously with, combining the polyelectrolytes in the solution. This approach can be taken to minimize or prevent premature coacervation and precipitation of the polyelectrolytes. In some embodiments, the combining step can comprise adding one of the polyelectrolytes to an aqueous solution comprising the oppositely charged polyelectrolyte and the chaotropic salt. For example, making a radiopaque embolic liquid in which one polyelectrolyte is in excess can comprise adding the oppositely charged polyelectrolyte to a solution comprising the excess polyelectrolyte, thereby avoiding a sign reversal of the overall charge in the solution during this step.

In some embodiments, the combining step can comprise mixing an aqueous solution comprising the polycationic polymer with an aqueous solution comprising the polyanionic polymer to create a radiopaque embolic liquid comprising the targeted amounts of each polyelectrolyte. One or both of the starting solutions can additionally include sufficient chaotropic salt to provide the appropriate salt concentration in the product solution.

The amounts of polyanionic polymer, polycationic polymer and chaotropic salt combined according the method can be selected based on the considerations discussed above. In some embodiments, the amounts of polycationic polymer and polyanionic polymer produce a total polyelectrolyte concentration in the solution of about 10% to about 30% w/v. In some embodiments, the amounts of the polyanionic polymer and the polycationic polymer can be based to produce a selected stoichiometric molar ratio in the radiopaque embolic liquid. This ratio may be theorized according to full dissociation of the ions in solution at a particular pH, e.g., neutral or physiological pH. In some embodiments, the polycationic polymer is present in excess. In some embodiments, the molar ratio of polycationic polymer to polyanionic polymer is about 1.05:1 to about 2.5:1. In some embodiments, the salt concentration is about 0.5 M to about 5 M.

In some embodiments, the method can comprise additional steps to adjust the solution's properties. For example, if the viscosity of the product solution is higher than desired, the solution may be diluted slightly (e.g., by addition of water) to decrease viscosity. In another case, if the combining step results in formation of coacervate complexes, further addition of salt to the solution can be employed to disperse the complexes. In some embodiments, making a radiopaque embolic liquid can comprise dissolving coacervate complexes of polyelectrolytes as discussed herein in a saline solution.

Methods of embolization using a radiopaque embolic liquid encompassed by the present disclosure can comprise introducing the solution into a site or structure to be embolized. In one embodiment, the radiopaque embolic liquid is used to treat arteriovenous malformation (AVM). In another embodiment, the radiopaque embolic liquid is used to treat aneurisms. Treatment of other conditions is also contemplated. In some embodiments, the site or structure to be treated is located in a blood vessel or other duct of a subject to be treated, particularly where said blood vessel or duct contains a flow of a physiological medium. Introduction of the radiopaque embolic liquid can be accomplished with a narrow gauge device such as a syringe, cannula, needle, catheter and the like. Accordingly, a kit for use in embolization can comprise a syringe containing an amount of a radiopaque embolic liquid described herein.

Once delivered to a subject and/or site or structure to embolized, the radiopaque embolic liquid can undergo coacervation and precipitate a hydrogel. Such coacervation can take place due to the radiopaque embolic liquid encountering a lower osmolality medium, such as blood plasma. For example, human blood plasma can have an osmolality of about 275-295 milli-osmoles per kilogram, which can be sufficiently low to cause coacervation and the formation of a hydrogel that can act as an embolic material. The resulting hydrogel can be detected as desired, as it can be radiopaque i.e., visible in CT scan or radiographic imaging.

### EXAMPLES

### Materials and Methods

### A. Preparation of exemplary radiopaque polyanionic polymer (RO acrylamide)

### 1. Polymerization process

The monomer is dissolved in water (25 - 60 °C) at concentration of 200 to 500 g/L. When necessary, pH is adjusted between 6 and 8 with diluted HCl solution or diluted NaOH solution. The monomer solution may also be slightly buffered with phosphate or acetate salts. The solution is filtered to remove insoluble impurities.

The solution is then transferred in a closed recipient (round bottom flask, twin or triple neck type) with adapted stirring system (moon shaped stirrer shaft on overhead stirrer). The solution is heated in an oil bath (double boiler) to temperatures between 50 to 80 °C. Argon bubbling is applied during heating and for at least 5 minutes before polymerization.

Polymerization is started with a radical initiator in 0.1% to 5% molar proportion based on pure monomer and preferably 0.1 to 1.0 mol%. Exemplary initiators that can be used include:
- Ammonium peroxodisulfate (APS)/N,N,N',N'-Tetramethylethylenediamine (TEMED) redox couple
- 2,2'-Azobis[2-(2-imidazolin-2-yl)propane] dihydrochloride (VA-044)
- 2,2'-Azobis(2-methylpropionamidine) dihydrochloride (V-50)

The initiator is dissolved in water in a known concentration and added to the monomer solution in weighed amount to reach the target molar ratio. When using ammonium peroxodisulfate (APS), a small volume of TEMED is added after approximately one minute. The usual volume is 100µL TEMED for 100 mg APS.

The polymerization is generally evidenced in the following minutes by an exothermic jump (+5 to 10 °C noticed on the temperature control) and an increased viscosity. Argon bubbling is then stopped, and heating under stirring is maintained for 0.75 to 8 hours depending on the initiator and the temperature applied.

In case of copolymerization with one or more water-soluble monomers, the process is unchanged. The co-monomers are mixed in the target proportion for dissolution.

Depending on the hydrophilicity of the monomer vs its polymer, additional salts may be added to prevent swelling after polymerization. This can be done with salinization (0.5 to about 2 M NaCl in the monomer solution). Similarly, for pH sensitive monomers, the solution may be buffered with 0.1 to 1 M phosphate or acetate salts.

In some cases, for slightly or non-water soluble monomers or co-monomers, a co-solvent (for example, ethanol, acetone, methanol, isopropanol, tetrahydrofuran, acetonitrile, dimethyl sulfoxide, methyl acetate, tertiobutanol, N-methylpyrrolidone, dioxane) can be used.

### 2. Purification

The polymer solution is diluted and dialyzed to remove unpolymerized monomers and impurities by different references of membrane. MWCO range from 1 k to 10 kDa. 100 - 150mL raw polymer solution are typically dialyzed in MWCO 3500 Da membranes against 10 L ultra-pure water for 3 to 5 days, replacing the water volume twice a day (morning / evening). The dialysis membranes are suspended above a 10 L beaker and immersed in water. A mild magnetic stirring is applied for homogenization in the water volume.

A faster purification can be achieved via tangential flow filtration using hollow fiber membranes in columns or cassettes. The purified polymer solution is then recovered for concentration through evaporation and then freeze-dried to collect the dry polymer.

### 3. Precipitation

**In** an alternate process, the polymer can be precipitated in 1 N hydrochloric acid. The polymer solution is added dropwise in the acid under strong agitation (6,000 rpm) using a dispersing instrument (IKA Ultra-Turrax). A minimum of 10 volumes of acid are used for 1 volume of polymer solution.

The polymer is collected through centrifugation and thoroughly rinsed with water before being evaporated on a sintered glass filter funnel and finally freeze-dried. The precipitation method accelerates the drying process for large volume of diluted polymer solution after dialysis. Indeed, it is not equivalent to a purification with dialysis. However, the polymer is dried in its acidic form and must be later dissolved in alkaline environment.

### 4. Characterization

The polymer is characterized in NMR ¹H spectroscopy in D₂O to assess effective polymerization, track possible residual monomer. Qualitative information is also provided by FT-IR spectroscopy to confirm polymerization. Aqueous gel permeation chromatography is also used to compare polymers with each other and evaluate molecular weight vs polystyrene sulfonate standards. Additionally, a light scattering detector can be coupled to concentration detectors in the chromatographic system that can provide absolute measurements of the molecular weight as desired.

### B. Preparation of polycationic polymer

Commercial products of cationic polymers and copolymers are available with different characteristics (average molecular weight, concentration and therefore viscosity, dry...) like polyDADMAC (polyquaternium 6), poly[2-(trimethylamine)ethyl methacrylate], Poly(ethyleneimine), Poly(2-vinyl-1-methylpyridinium bromide), poly(acrylamide-co-diallyldimethyl ammonium chloride) (= polyquaternium 7) etc.

Commercial solutions in water could be concentrated by solvent evaporation or freeze-dried to recover the dry polymer and prepare custom solutions. When starting from the monomer(s) the polymerization or copolymerization process is very similar to that described for the polyanionic polymer (see Examples). Purification methods are the same.

Polycationic polymers are also characterized using the techniques previously mentioned.

### C. Preparation of polyelectrolyte solution

### 1. Direct blending

The preparation of the polyelectrolyte solution consists in the mixing of the oppositely charged species in a salty medium avoiding early coacervation of the complex.

### 2. Precipitation and dissolution

Another method consists in direct coacervation in water or low concentration saline solution of the two polyelectrolytes in proper ratio. The polymer concentration should not be too high for a better homogenization. The precipitate can then be collected and washed from its impurities-especially the excess polyelectrolyte that does not participate into the gel-and further dissolved in a solution with a sufficient ionic strength (salt concentration).

### 3. Exemplary parameters

The ratios are calculated based on the molar weight of the monomers or repeating pattern in the polymer. Examples of molar ratios for various polycation/poly(RO Acrylamide) couples and polycation/poly(styrene sulfonate) sodium salt couples are provided in Table 4:

**Table 4. Exemplary molar ratios of polycation / poly(RO Acrylamide) and polycation / pSSNa couples (mol:mol)**

| Polycation | poly(DEAE Acrylamide) | poly DADMAC | Poly METAC | Poly Quarter-nium-2 | ε - poly(L-lysine) | Poly(ethylene imine) | Poly(VBT MAC) |
|---|---|---|---|---|---|---|---|
| molar ratio with poly (RO acryl-amide) | 1.9 : 1 | 2.0 : 1 | 2.1:1 | 1.05 : 1 | 2.1 : 1 | 1.75 : 1 | 2.0 : 1 |
| molar ratio with poly(styrene sulfonate) sodium salt | 2.0 : 1 | 1:1 | 1.3 : 1 | 1 : 2.4 | 1 : 1.05 | 1.7 : 1 | 1 : 1 |

Table 5 below shows exemplary NaCl and CaCl₂ concentrations for stable solutions of several polycations + poly(RO acrylamide) couples depending on the polymer concentration. These results are based on numbers of observations involving several polymers batches (either polyanion and polycation) and various molecular weights.

**Table 5. NaCl and CaCl₂ trigger concentrations according to polycation and global concentration.**

| Polycation | Molar ratio with poly(RO acrylamide) (mol : mol) | Global polyelectrolytes concentration (w/v) | NaCl trigger concentration (mol/L) | CaCl₂ trigger concentration (mol/L) |
|---|---|---|---|---|
| **PolyDADMAC** | 2.2 : 1 | 30 - 35% | 0.7 - 0.9 | |
| | 2.2 - 2.6 : 1 | 20 - 30% | 0.9 - 1.5 | |
| | 1.9 - 2.1 : 1 | 20 - 30% | 1.5 - 1.8 | 0.5 - 0.7 |
| | 1.9 - 2.0 : 1 | 15.6% | 1.4 | 0.44 - 0.57 |
| **polyDEAE** | 1.9 : 1 | 20 - 25% | 1.7 - 2.0 | 0.5 - 0.65 |
| | 1.9 : 1 | 15.4% | 1.6 | 0.5 - 0.75 |
| **polyMETAC** | 2.0 - 2.2 : 1 | 20 - 25% | 1.1, - 1.3 | 0.6 - 0.7 |
| | 2.1 : 1 | 15.8% | 0.8 - 1.0 | 0.35 - 0.4 |
| | 2.1 : 1 | 10 - 15% | 1.3 | 0.3 - 0.4 |
| **Polyquaternium-2** | 1.05 : 1 | 29% | | 0.3 |
| | 1.05 : 1 | 15.6% | | 0.55 |

### Example 1: Preparation of dry poly(RO acrylamide)

A 100 g quantity of radiopaque monomer N-amido-triiodoisophtalic acid butanamido-acrylamide sodium salt ("RO acrylamide") was dissolved in 270 mL ultra-pure water at 60 °C with a magnetic stirrer. The solution was purified by centrifugation at 3000 rpm for 5 min to remove the largest insoluble impurities and then filtered with a sintered glass funnel. All insoluble matter was gathered and put to dissolve again in 30 mL water at 80 °C under magnetic stirring. The resulting suspension was filtered again the same way and the clear liquid phases were gathered in a triple neck type round bottom flask. The pH of the solution was 6.5.

A moon-shaped PTFE stirrer shaft was mounted on the recipient flask as well as temperature control probe and a tube for argon bubbling. The solution was heated to 70 °C in an oil bath (double boiler) under argon bubbling and 200 rpm agitation with an overhead stirrer for 5 min before the addition of initiator. Heating was temporarily stopped to observe exothermicity during polymerization. A solution of 352 mg ammonium peroxodisulfate (APS) in 4.12 g water (8.5 wt%) was prepared and 3.52 g of this solution (i.e. 301 mg APS) was quickly added to the medium. This caused the temperature to drop down to 67 °C.

The concentration of the monomer solution was about 30% w/v and the molar ratio of initiator to pure monomer was 1.07 mol%. One minute later, 300 µL TEMED was added and the temperature increased to 77 °C during the following minutes.

Heating at 70 °C was applied again for 1 hour under stirring; argon bubbling was cut several minutes after the temperature rise. The polymer solution was then diluted and transferred in a large dialysis sack with MWCO 3500 Da.

After five dialysis sessions against 10 L ultra-pure water over 2.5 days, the solution was divided into two smaller volumes in new dialysis sacks with MWCO 3500 Da, both for five other sessions against (2x) 10 L water over 3 days.

The polymer solution volumes were then gathered for evaporation at room temperature under an extraction hood. Once the solution concentrated to a volume about 200 mL, the polymer was precipitated dropwise in 1 N HCl, 8 to 10 mL in 150 to 200 mL fresh acid under vigorous stirring at 6,000 rpm with an Ultra Turrax disperser. The microparticles formed were collected through centrifugation and thoroughly rinsed with water before evaporation on a sintered glass filter funnel and finally freeze-dried. 5.57 g of pure RO polymer in its acidic form was recovered. The overall yield was about 6.5 % based on pure monomer.

### Example 2: Preparation of poly(RO acrylamide) solution without acidic precipitation

18 g of N-amido-triiodoisophtalic acid butanamido-acrylamide sodium salt ("RO acrylamide") were dissolved in 50 mL ultra-pure water at 60 °C with a magnetic stirrer.

Insoluble impurities were filtered on a sintered glass funnel. All insoluble material was gathered and put to dissolve again in 10 mL water at 60 °C under magnetic stirring. The resulting suspension was filtered again the same way and the clear liquid phases were gathered in a Shlenk tube. The pH of the solution was about 6.5.

The tube was immersed in an oil bath and heated to 60 °C (temperature control in the solution). Agitation was set to 500 rpm with a magnetic stirrer and under argon bubbling. As described in Example 1, heating was temporarily stopped during initiator addition (1.286 g APS solution 4.32 wt% equates to 54 mg initiator) followed by 60 µL TEMED. The temperature rose to 75 °C and an increase of viscosity was observed through the agitation and the size of argon bubbles in the medium.

Heating was turned back on for 3 hours under stirring in closed tube after argon flushing. The solution was then transferred and let to cool down and partially evaporate overnight under extraction hood. A sample was freeze-dried without purification and sent for NMR analysis.

The polymer solution was after diluted and filtered again on a sintered glass funnel before dialysis in a MWCO 3500 Da sack against 4.5 L in 6 sessions for 3 days. After evaporation, the resulting syrup was freeze-dried and 4.32 g polymer was collected. Overall yield = 26.3% (NMR sample not included).

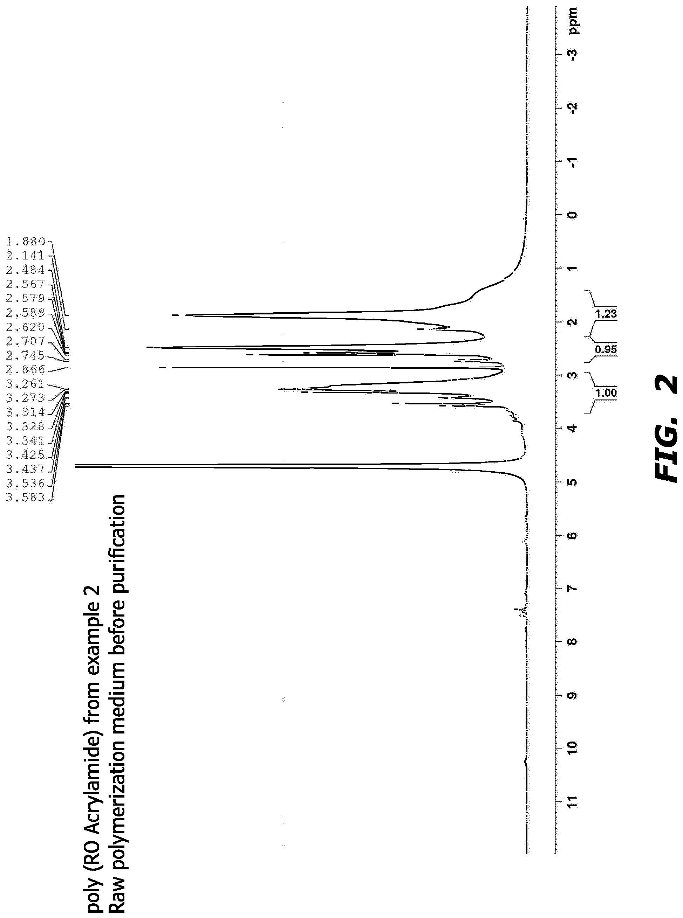

The NMR spectrum of the raw polymerization medium (FIG. 2) revealed possible monomer traces (vinylic protons 6.1; 5.7 ppm) suggesting that the polymerization was not complete. This could explain the lower yields with this monomer.

The purified polymer was dissolved in 12.55 g at 50 °C. The pH was adjusted from 4.7 to 7.95 with several drops of 4 N NaOH. The density of this solution was 1.149 at room temperature (RT) and the concentration was evaluated to 27 wt% based on dry weight after evaporation of 1mL.

### Example 3: Preparation of poly(RO acrylamide) with azo initiator VA-044

The method described in Example 2 was reproduced on 11 g RO acrylamide in 48 mL water after filtration. Polymerization was performed at 50 °C for 21 h with 32 mg 2,2'-azobis[2-(2-imidazolin-2-yl)propane] dihydrochloride previously dissolved in 3 mL water. Monomer solution concentration was 21 % w/v and initiator/pure monomer molar ratio was 0.73 mol%.

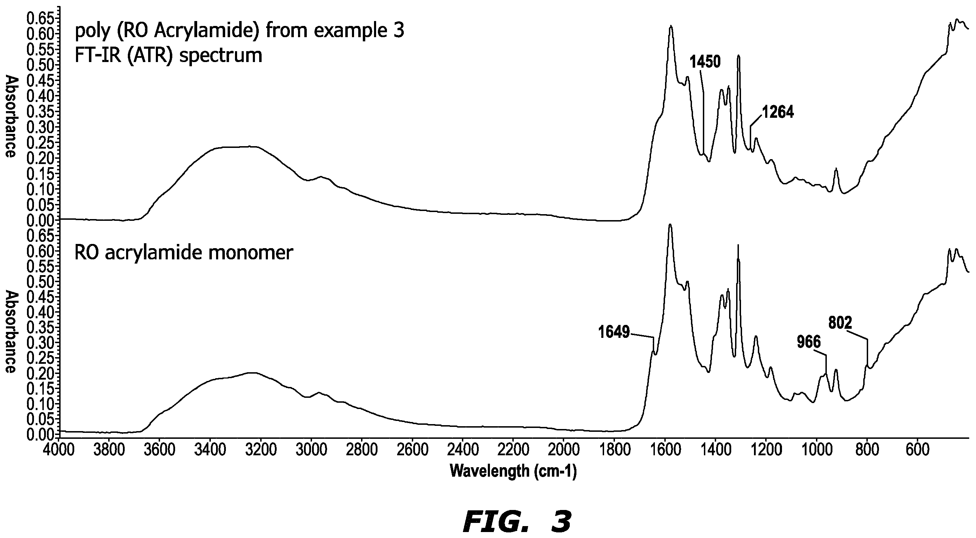

The polymer was dried unpurified and analyzed via FT-IR spectroscopy, the results of which are shown in FIG. 3. After comparison with the monomer and other polymers obtained with similar protocols to Examples 1 & 2, polymerization could be evidenced by the disappearance of the absorption band around 1650 cm⁻¹ attributed to a C=C elongation in the monomer. A peak complex was also significantly attenuated in the 1020 - 940 cm⁻¹ area and one band around 800 cm⁻¹ attributed to C-H deformations out of plan in vinyl motif. An additional band appeared around 1265 cm⁻¹ that may match with a vibration mode specific to methylenes in a polymer backbone that is sometimes confirmed by a (weak) signal around 1450 cm⁻¹ (δ_{C-H} in plan in CH₂).

### Example 4: Preparation of poly(RO acrylamide) with azo initiator V-50

The method described in Example 2 was reproduced on 10.9 g RO acrylamide in 40 mL water after filtration. Polymerization was conducted at 50 °C with 30 mg 2,2'-azobis(2-methylpropionamidine) dihydrochloride previously dissolved in 2 mL water. Monomer solution concentration was about 24% w/v; initiator/pure monomer molar ratio was 0.73 mol%.

The temperature was increased to 60 °C 10 min after addition of the initiator and maintained for 19 h. Argon bubbling was maintained for about 3 h after addition of the initiator.

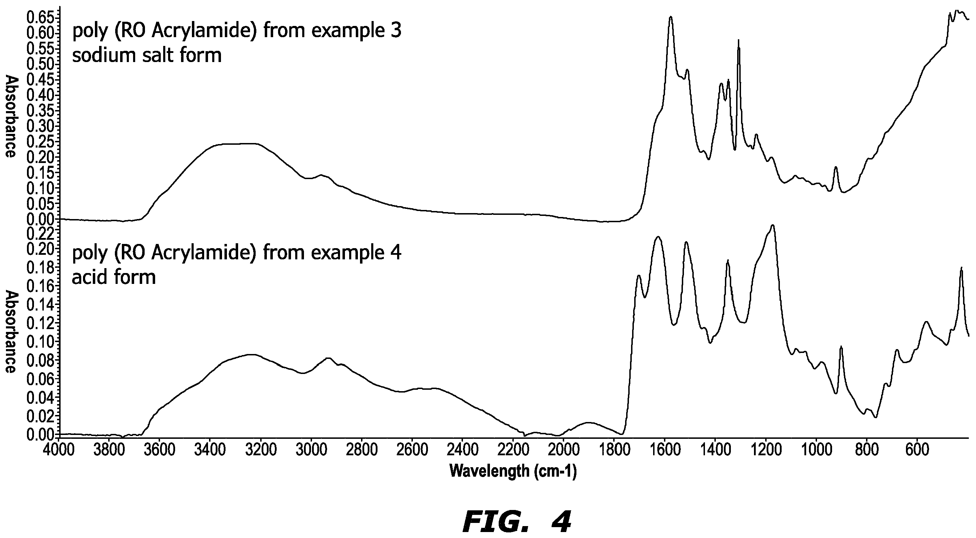

The polymerization medium was then concentrated via evaporation and precipitated in 1 N HCl, rinsed with water and subjected to evaporation following the protocol described in Example 1. After drying, 7.48 g of unpurified polymer in its acidic form was collected. An FT-IR spectrum of the acidic polymer showed many differences with that of the sodium salt (FIG. 4).

### Example 5: Preparation of poly(RO acrylamide) solution after acidic precipitation

The method described in Example 2 was reproduced on 11.16 g RO acrylamide dissolved at 60 °C in 40 mL water after filtration. The pH of the solution was 6.75.

The initiator ammonium peroxodisulfate (2.18 wt% in an aqueous solution) was sequentially added to the monomer under heating (50 °C), stirring (300 rpm) and argon bubbling. First, 1.13 g APS solution was added followed by 50 µL TEMED. Then 0.11 g APS solution was added 10 min after the first addition and heating was increased to 60 °C 3 hours later. Finally, 1.2 g APS solution was added for a total of 52 mg initiator followed by 50 µL TEMED. Total molar ratio of initiator to pure monomer was 1.66 mol%.

The stirring speed was raised to 500 rpm for better homogenization; heating at 60 °C was extended a further hour. The polymerization medium was then concentrated via evaporation and precipitated in 1N HCl, rinsed with water and spined following the protocol described in Example 1.

After drying, the solid was crushed with a mortar and a pestle. The powder (7.03 g) was slowly dispersed in 18.2 mL 0.25 N NaOH solution under magnetic stirring. As the pH was still < 2 the polymer did not dissolve. The pH was raised with additions of pure sodium hydroxide pellets up to 7.5. The viscosity of the solution dramatically increased so that it was necessary to dilute it to a concentration of 22.4 wt%. After sample collection, 3.6 g NaCl was dissolved in the solution and insoluble impurities were removed by centrifugation. The concentration of the final solution was determined at 19.8 wt% RO polymer, 2.91 M NaCl, having a pH of 7.13 and a specific gravity of 1.23.

### Example 6: Preparation of polyMETAC polycation

13.31 g [2-(Methacryloyloxy)ethyl] trimethylammonium chloride (75.2 wt% aqueous solution) was diluted with 31.77 mL water in a Shlenk tube with a magnetic stirrer. The monomer concentration was lowered to about 20.8 wt%, for previous experiments had shown a viscosity jump during polymerization in more concentrated media together with a loss of fluidity that hampered homogenization.

The tube was immersed in an oil bath and heated to 60 °C (temperature control in the solution). Agitation was set to 500 rpm under argon atmosphere. As described in Example 1, the heating was temporarily stopped during initiator addition (100 mg APS solution in 3 mL water).

Immediately after addition of 100 µL TEMED, the temperature began to rise by up to 9 °C and viscosity increased, but the medium was still fluid enough for a correct agitation. Heating was turned back on for 2 hours at 60 °C under stirring in closed tube after argon flushing.

After reaction and cool down, the solution was transferred and diluted in an MWCO 3500 Da dialysis sack for 4 sessions against 4.5 L water and then concentrated by evaporation and then freeze-dried.

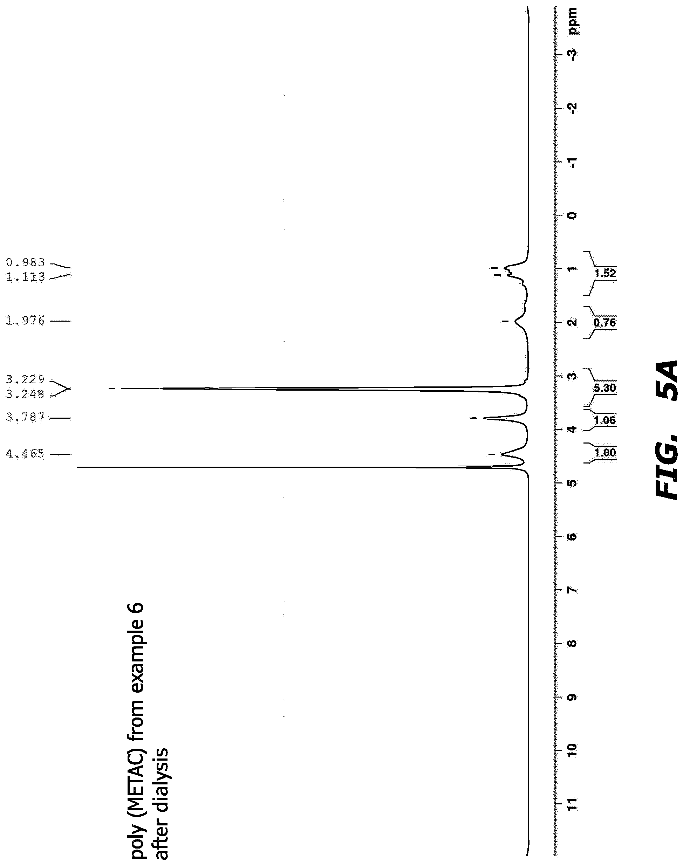

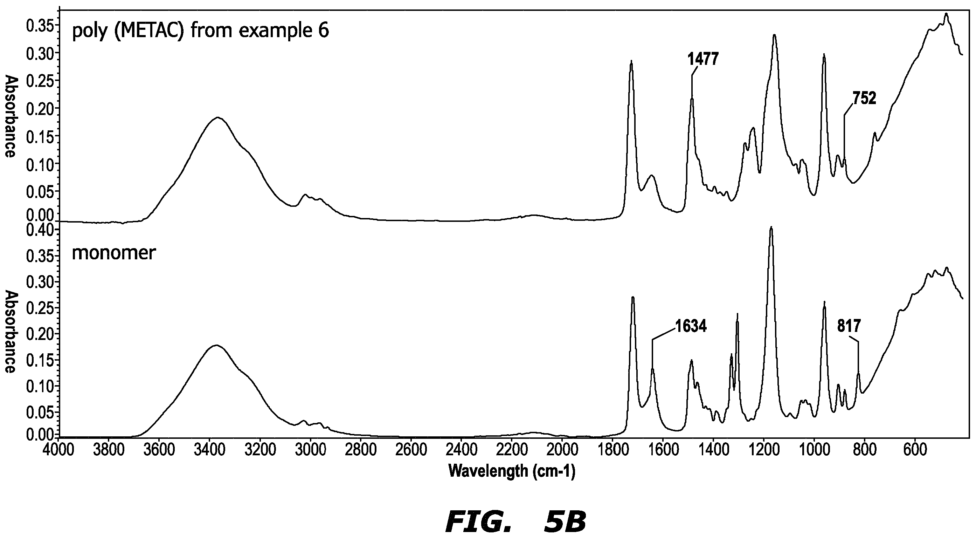

Dry samples were analyzed in NMR and FT-IR spectroscopy. Polymerization was assessed on NMR spectrum (FIG. 5A) by the absence of vinylidene protons (δ 6.05 and 5.56 in the monomer) and disappearance of the signal at 817 cm⁻¹ in the FT-IR spectrum compared to the monomer (FIG. 5B). Some additional clues in the FT-IR spectrum suggested the formation of a polymeric chain (increased absorbance at 1477 cm⁻¹ and appearance of a peak at 752 cm⁻¹ both related to methylene).

### Example 7: Preparation of poly(METAC-co-HEMA) copolymer solution

The molar ratio between the two monomers METAC and 2-hydroxyethylmethacrylate (HEMA) was varied in the following proportions: 50:50; 70:30; 80:20; and 90:10 METAC:HEMA. The preparation of the 80:20 copolymer is described below:

8.33 g METAC in an 75.2 wt% aqueous solution was added to a solution of 1.013 g HEMA stabilized in 23.13 g ultra-pure water with 2.246 g sodium chloride for ionic strength. The pH was adjusted to 6.09 with diluted NaOH.

The resulting solution was transferred in a Shlenk tube. The tube was immersed in an oil bath and heated to 65 °C (temperature control in the solution). Agitation was set to 500 rpm with a magnetic stirrer and under argon atmosphere.

Heating was temporarily shut down during the addition of the initiator (2.204 g APS, 3.18 wt% solution) followed by 90 µL TEMED. The stirring speed was raised to 1000 rpm. A slow and discrete increase of the temperature was observed (+5 °C) and argon bubbling was stopped. The concentration of the total monomers in the medium was 19.7 wt%. The salt concentration was 1.06 M NaCl and the molar ratio of initiator to pure monomers was 0.81 mol%.

After 1 h at 65 °C, the solution was allowed to cool down and transferred to a MWCO 3500 Da dialysis sack for 5 sessions against 4.5 L water. The purified solution was then freeze-dried and 1.88 g dry copolymer was collected for a global yield of around 26%.

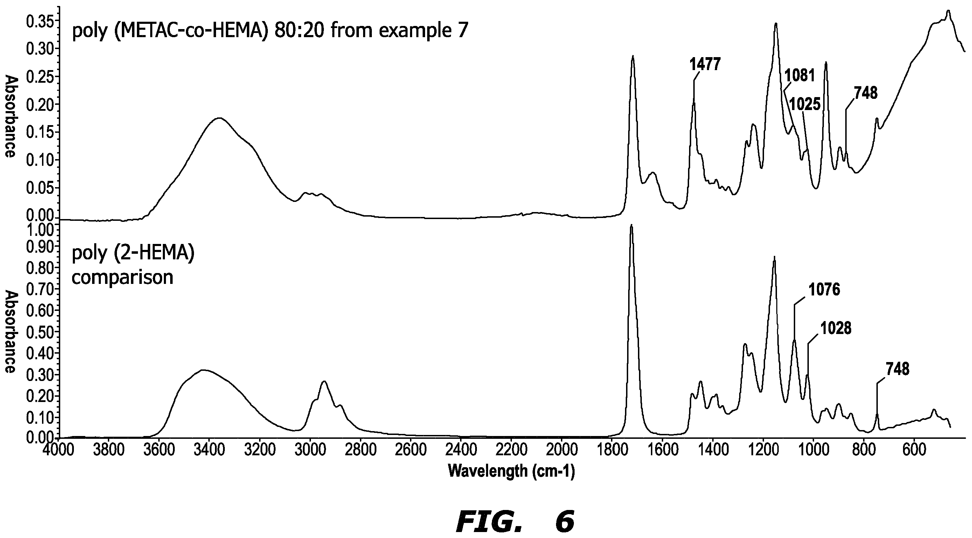

Polymerization was assessed in FT-IR spectroscopy (FIG. 6) compared to the monomers spectra by the disappearance of the signal at 820 cm⁻¹, increased absorbance at 1481 cm⁻¹ and appearance of a peak around 750 cm⁻¹. The presence of HEMA moieties in the polymer was evidenced by an additional band at 1081 cm⁻¹ and an extra absorbance at 1025 cm⁻¹ matching respectively with 1076 and 1026 cm⁻¹ bands (*v*_{C-O} ester/alcohol in polyHEMA).

The copolymer was dissolved in water, yielding in an ultra-viscous clear solution with a concentration of 33.3 wt% total polymers, a specific gravity of 1.05 and a pH = 8.5. The proportion of the polycation motif in the solution was around 1.4 mmol/g.

### Example 8: Preparation of poly(RO acrylamide-co-AMPS) polyanion

The preparation of a 2:1 n:n poly(RO acrylamide-co-AMPS) copolymer is described below:

2.63 g 2-Acrylamido-2-methylpropanesulfonic acid was first dissolved in 50 mL ultra-pure water (pH of the solution = 0.9). As this solution was highly protic, the RO Acrylamide monomer may be partially protonated when added to the AMPS acidic solution and flocculated. This can be addressed by forming the AMPS sodium salt before mixing it with the RO Acrylamide monomer; this was done by addition of 3 sodium hydroxide pellets, raising the pH to 12.6.

19.58 g RO acrylamide monomer was then gradually added to this solution and pH was stabilized to 6.75 by careful additions of 1.5 mL 1 N HCl. The solution was diluted and heated to 60 °C before filtration on a sintered glass funnel by small successive volumes with vacuum suction.

The polymerization was carried out in a 150 mL beaker with mechanical overhead stirring (3 blades PTFE propeller 500 rpm), a double boiler oil bath and argon bubbling. The solution was heated to 62 °C, with heating temporarily shut down during the addition of APS solution (105 mg in 5 mL water) followed by 80 µL TEMED. A viscosity jump was soon observed and a discrete temperature elevation (+3 °C). Heating was turned back on for 1.5 hours at 60 °C under stirring with gentle argon bubbling.

The concentration of the polymers in the medium was 22.3% w/v. Total molar ratio of initiator to pure monomers was 1.26 and the theoretical molar ratio between monomers was 2:1 RO acrylamide:AMPS. A sample was freeze-dried at this stage for NMR analysis.

After drying, about 22 g of the raw copolymer was redissolved in 51 g water at 60 °C. Dissolution was complete and it was further diluted for dialysis in MWCO 3500 Da sack against 4.5 mL water. However, the solution was still too much concentrated and the osmotic pressure on the dialysis membrane became too strong after several hours so it was decided to dilute it again and place it in a larger dialysis sack for 10 sessions against 10 L water. After final evaporation and freeze-drying, 2.76 g product were collected for an overall yield of 13.5%.

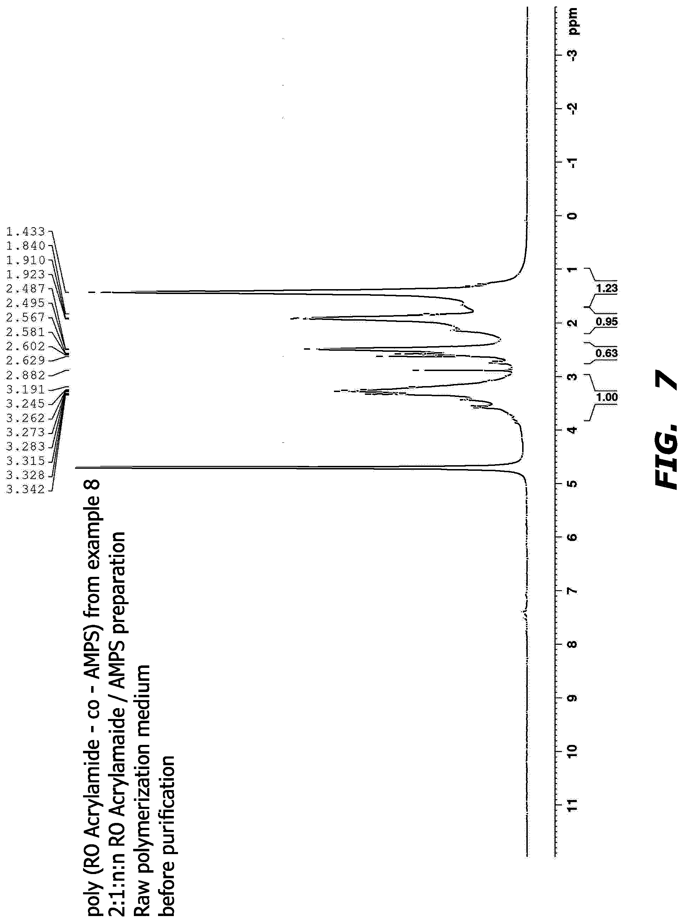

The absence of signals above 5.5 ppm in the NMR spectrum of the raw polymerization medium (FIG. 7) suggest the consumption of the monomers and good polymerization rate.

### Example 9: Preparation of poly(RO acrylamide-co-styrene sulfonate) polyanion

2.69 g sodium 4-vinylbenzene sulfonate (96.0%) and 5.48 g RO acrylamide monomer (91.3%) were dissolved in 20 mL ultra-pure water at 60 °C. The pH of the resulting solution was 7.9. This solution was centrifuged and filtered to remove insoluble and transferred in a Shlenk tube with a magnetic stirrer. The tube was immersed in an oil bath and heated to 70 °C (temperature control in the solution) under argon atmosphere. Agitation was set to 500 rpm.

After 15 min at 70 °C with argon bubbling, heating was interrupted and 489 mg of a solution of 9.57 wt% VA-044 initiator in water were added to solution. During the 5 - 10 min after addition, a slight increase of temperature (+ 4 °C) was observed and a probable viscosity rise evidenced by the size of argon bubbles forming in the liquid medium. Heating was turned back on for 4 hours at 70 °C under stirring in closed tube after argon flushing. The reacting medium was then allowed to cool down overnight under stirring. The concentration of the polymers in the medium was 30.0% w/v. Total molar ratio of initiator to pure monomers was 0.75 mol% and the theoretical molar ratio between monomers was 35:65 RO acrylamide:sodium styrene sulfonate.

The polymer solution was after diluted before dialysis in MWCO 3500 Da sack against 10 L in 9 sessions for 5 days. The dialysate fluid was concentrated by evaporation and freeze-dried.

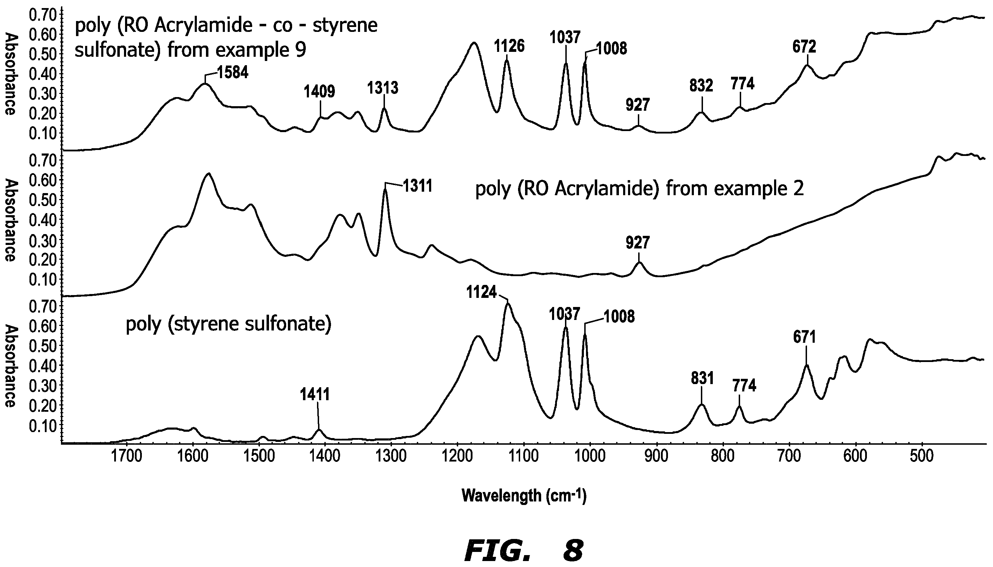

As shown in FIG. 8, polymerization was evidenced in FT-IR spectroscopy especially by the disappearance of the absorbance bands between 950 and 1020 cm⁻¹ and another at 805 cm⁻¹ attributed to deformations of the C-H ethylene bonds in the monomers. Characteristic absorption bands of the two polymers were present and the contribution of each polymer to the spectrum was evaluated by the means of a bicomponent research with RO acrylamide polymer from Example 2 as a known component. The higher match value for the second component above all was 75% for a commercial reference of sodium polystyrenesulfonate and the composite values indicating the amount of each component in the spectrum were coincidentally, 36.3:63:7 % poly(RO acrylamide):poly(sodium styrene sulfonate).

The copolymer was characterized in aqueous gel permeation chromatography (GPC) and showed a single peak with a retention time > RO acrylamide homopolymers or copolymers with AMPS suggesting lower molecular weights, but peak shape also indicated a probable effective copolymerization between these two monomers unlike the RO acrylamide-co-AMPS for which a blend of polymers was suspected.

### Example 10: Comparison of (RO Acrylamide) polymers by GPC

Several RO acrylamide polymers and copolymers were compared in aqueous gel permeation chromatography (size exclusion) along with the native monomer. The aromatic nuclei in the molecules provide absorption in UV wavelengths (200 - 254 nm) for detection. This method was helpful to evaluate the molecular weight according to poly(styrenesulfonic acid) sodium salt standards and polydispersity of the polymers synthesized but also their purity especially regarding the residual monomer.

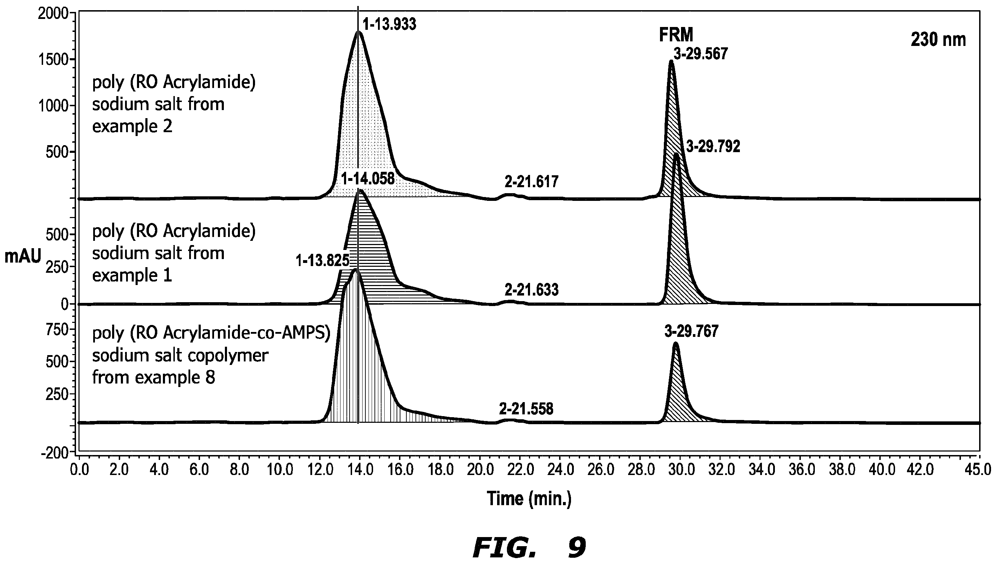

FIG. 9 shows polymers described in Examples 2, 1 and 8 (top to bottom). It was found that the average molecular weights were close. In this system, the retention time around 14 min was between a 2,070,000 Da poly(styrenesulfonic acid sodium salt) analytical standard eluted in 13.7 min and a 145,000 Da poly(styrenesulfonic acid sodium salt) analytical standard eluted in 15.7 min. The lower mass impurity eluted in 21.6 min was present in larger amount in the monomer, it matches with a 4000 Da molecule when compared to the poly(styrenesulfonic acid sodium salt) standard calibration and the intensity of the signal gives information on the efficacy of the purification process.

This method also provided valuable information by comparing the elution times and profiles of the polymers synthesized with different initiators or different amounts of a same initiator (data not shown). A set of three consecutive aqueous and polar organic GPC/SEC columns were used for optimized separation of the molecular weights. The mobile phase was 95% phosphate buffer 0.1 M pH 8,75 + 0.1 M NaCl / 5% Methanol v/v; 0.8 mL/min. Flow rate marker (FRM) was p-toluenesulfonic acid sodium salt.

### Example 11: Preparation of a polyelectrolyte solution poly(RO Acrylamide) + polyDADMAC

2.038 g of the poly(RO Acrylamide) solution prepared as described in Example 5 was mixed under stirring with 1.522 g of a commercial solution of 20.8 wt% polyDADMAC (pH = 7.1). As the polyanion solution was already salted with 2.91 M NaCl, precipitation was avoided.

The viscosity and salinity were carefully lowered with addition of 0.448 g water and a viscous, slightly turbid orange-pink liquid was obtained with a concentration of 20.2% w/v total polymers and a final salt concentration of 1.35 M. The molar ratio of polycation to polyanion was evaluated to 3.6:1, and pH = 7.4.

This solution was injected through a 1 mL syringe and a microcatheter (0.028" ID) with length shortened to 20 cm in a 8.5 cm diameter Petri dish filled with saline solution 0.9% NaCl (pH 7.4). The liquid slowly hardened after spreading for a few seconds on the bottom of the dish. In the same time it became white.

### Example 12: Preparation of a polyelectrolyte solution(RO Acrylamide) + polyDADMAC by dissolution of the polyelectrolytes complex

Specific amounts of the poly(RO Acrylamide) solution prepared as described in Example 5 and commercial solution of 20.8 wt% polyDADMAC in excess were mixed together under stirring and a large amount of water was poured into the mix. This resulted in the precipitation of the polyelectrolyte complex in a pinkish glue. The supernatant was removed and it was found that it contained a small amount of polyDADMAC but no evidence of the polyanion.

The gel was thoroughly rinsed with water to remove salt and excess polycation and then dried. One g of the resulting solid was put to dissolve in 3 mL of a 5 M hypersaline solution. After a fairly advanced dissolution 5 hours later, the medium was slowly diluted by dropwise additions of 2 mL water. The solution was still slightly turbid and highly viscous and was diluted with 0.5 mL water before filtration on a nylon syringe filter 1 µm. The polyelectrolytes concentration was 18.8% w/v in a salt concentration of 2.8 M, specific gravity = 1.13.

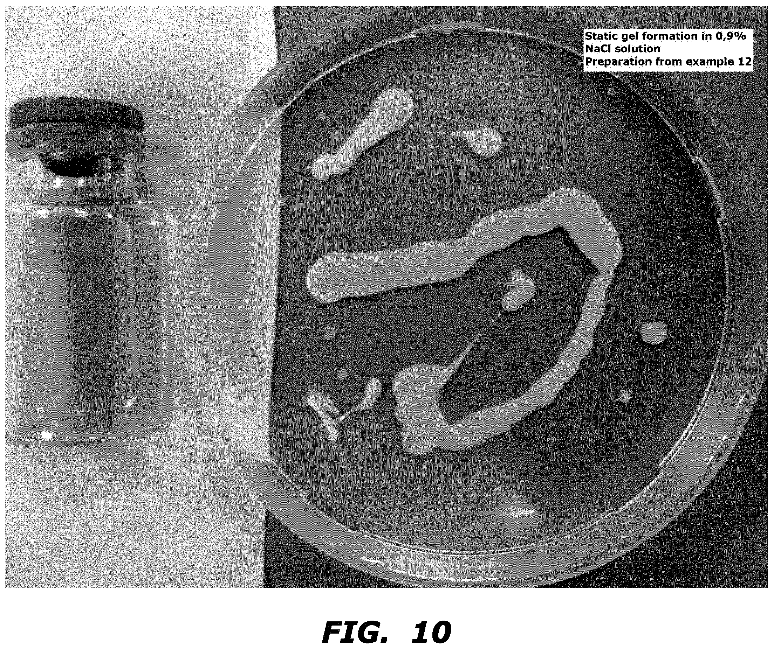

This solution was injected through a 1 mL syringe and a microcatheter (0.028" ID) with length shortened to 20 cm in a 8.5 cm diameter Petri dish filled with saline solution 0.9% NaCl (pH 7.4). As shown in FIG. 10, the liquid did not spread as much in the Petri dish but took about 30 s to harden and whiten. However, after complete diffusion of the salt the gel was cohesive and non-flowing. It appeared later that higher salt concentrations slow the hardening process and optimal polyelectrolyte ratios give stronger gels. Premature precipitates are purer, but involve high salt concentration for complete dissolution.

### Example 13: Preparation of highly concentrated polyelectrolyte solution (RO Acrylamide) + polyDADMAC

A commercial solution of 20.8 wt% polyDADMAC was concentrated through evaporation and freeze-dried. A new solution was prepared with the dry polymer with a concentration of 40 wt% and a pH adjusted to 7.6. To 0.592 g of this solution, 46 mg NaCl was added and dissolved under magnetic stirring. Then 1.826 g of the solution of poly(RO acrylamide) prepared as described in Example 2 was slowly added dropwise under magnetic stirring. Large flocs of polyelectrolyte complex coacervated and were dispersed with addition of 41 mg of NaCl dissolved in the liquid phase.

A solution that was highly viscous, yet able to be aspirated in a syringe, was obtained with a concentration of 34% w/v total polymers, a polycation/polyanion ratio of 2.2:1 n:n and a salt concentration = 0.7 M NaCl; pH was about 6.6. The main issue with highly viscous solutions was the air bubbles trapped in the volume that could only be removed by centrifugation.

This solution was injected through a 1 mL syringe and a microcatheter (0.028" ID) with length shortened to 20 cm in a 8.5 cm diameter Petri dish filled with saline solution 0.9% NaCl (pH 7.4). The pressure required to eject the solution through the catheter was deemed too high for the expected application, not only because of the viscosity but also because the gel quickly formed in the microcatheter previously flushed with saline solution. The gel extruded from the catheter immediately hardened without spreading and was rigid enough to be grabbed using tweezers without tearing.

Similar preparations with reduced concentration and minimal salinity suggested the feasibility of a concentration between 20 and 30% w/v for this couple of electrolytes in 2.2:1 molar ratio.

### Example 14: Preparation of polyelectrolyte solution (RO Acrylamide) + polyDADMAC for kidney model embolization in a dynamic flow

In 0.9985 g of a concentrated solution of 40 wt% polyDADMAC, 146 mg NaCl was dissolved under magnetic stirring. Then 3.074 g of the solution of poly(RO acrylamide) prepared as described in Example 2 was slowly added dropwise under magnetic stirring. A small volume (0.378 g) of hypersaline solution 5 M NaCl was then added to slightly increase salinity. The last flocs of coacervated polyelectrolyte complex were subsequently dispersed.

Polymer concentration was set to about 30% w/v and NaCl concentration to about 1 M with a last volume of water (0.246 g) added dropwise with a view to lower the viscosity. At each drop of water, a thin film of coacervate formed at the surface of the solution and dissolved shortly after under stirring. The molar ratio of polycation to polyanion was 2.21:1. This solution was easily aspirable in syringes and air bubbles trapped could be properly evacuated after suction.

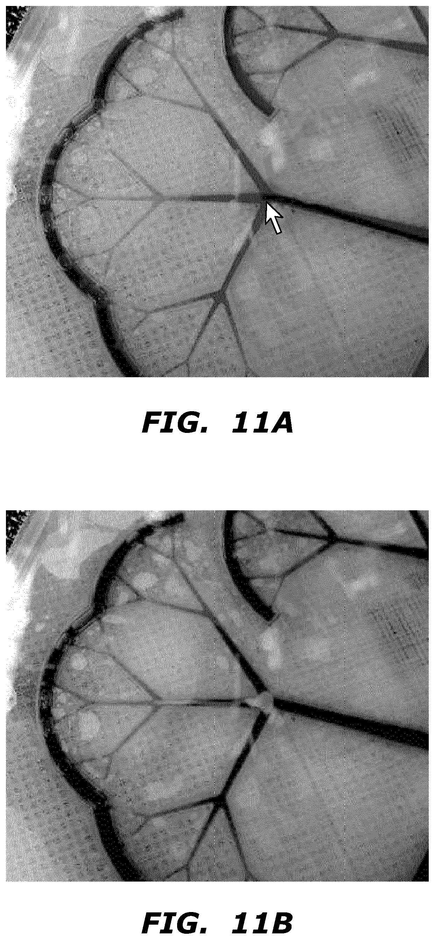

The kidney model was a hermetic 3-D printed device simulating blood circulation in a vascular network ranging from 0.3 to 2.4 mm internal diameter. Blood was simulated with saline solution of 0.9% NaCl + 0.04% w/w Remazol Brilliant Red F3B dye adjusted to pH 7.10. The blood flow was simulated by a peristaltic pump set at 250 mL/min. 0.5 mL of the solution was delivered in the model via a 130 cm 2.7 F microcatheter. The chronological frame is presented in FIG. 11A through FIG. 11D.

FIG. 11A shows the catheterization at a vascular site corresponding to 1.4 mm inner diameter (the arrow indicates location of the microcatheter tip).

FIG. 11B shows just after injection had begun. The gel immediately formed, was briefly diffused under the flow and hardened in a few seconds.

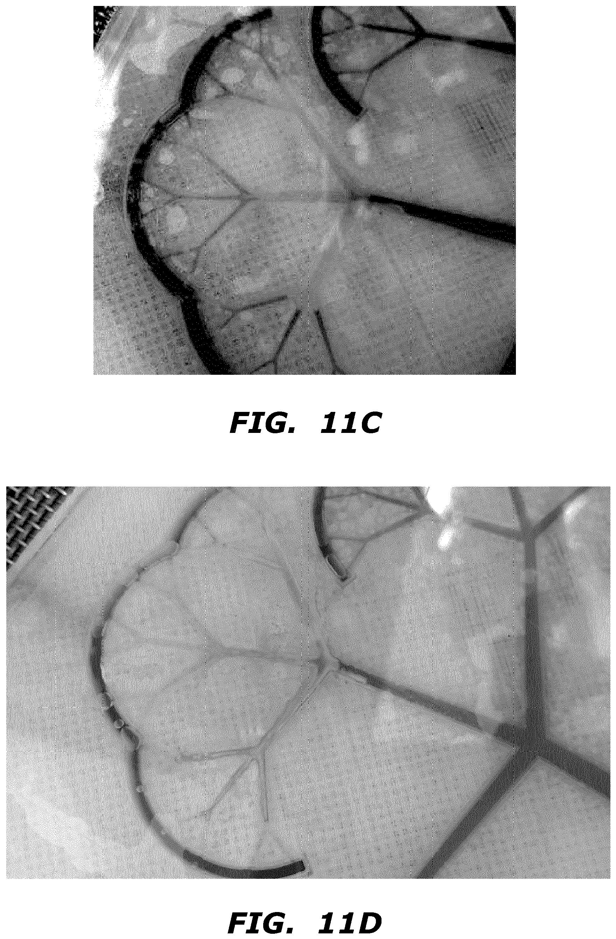

At the time shown in FIG. 11C, liquid embolic delivery stopped and the catheter was easily removed from the "embolization site". The jelly material didn't stick to the tip of the device and filled 3 channels with inner diameter shrinking down to 0.8 mm or a bit less.

The 'kidney' model was then rinsed with clear water after the procedure and fed again with colored saline to visualize the obstruction. As shown in FIG. 11D, the flow was interrupted downstream of the occlusion site (smaller channels stayed clear) in contrast with free channels (visible in the upper right of the picture).

### Example 15: Preparation of a polyelectrolyte solution poly(RO Acrylamide) + polyMETAC

The polymer obtained in Example 3 was dissolved to a concentration of 20.9 wt% in a 2.1 M saline solution. 1.518 g of this solution was mixed with 1.22 g solution of 15.8 wt% polyMETAC in water.

The polycation was obtained following a process very similar to that described in Example 6, starting with the same raw material. The polymerization was done in twice more concentrated solution and dialyses were performed with 12 -14 kDa MWCO membranes.

A partial macroscopic coacervation was observed at a 1.06 M saline concentration that dispersed a few minutes later under magnetic stirring resulting in a clear orange-pink viscous solution with a total PEC concentration = 21.2% w/v; pH = 7.05. The molar ratio of polycation to polyanion was 2.17:1.

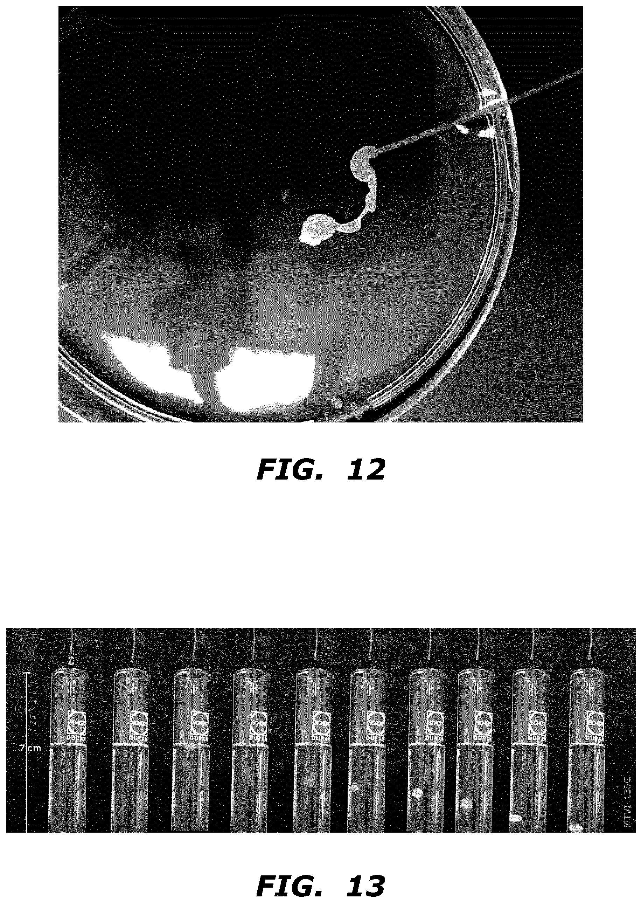

The PEC solution was injected through a 1 mL syringe and a 0.028" ID microcatheter with length of 130 cm in a 8.5 cm diameter Petri dish filled with 0.9% NaCl saline solution at pH 7.1 (see FIG. 12). Once diffused in the low osmolar medium, the PEC solution quickly hardened in a cohesive gel--still a bit fluid--that could be grabbed using tweezers immediately after extrusion. Solidification of one drop of this solution was measured in a 700 ms time-lapse image. As shown in FIG. 13, gelation of this solution occurred in a period less than 1 s.

### Example 16: Preparation of a polyelectrolyte solution poly(RO Acrylamide) + poly(METAC-co-HEMA)

The poly(RO Acrylamide) solution obtained in Example 2 was mixed with several polycations in comparison experiments. The 80:20 n:n solution of cationic copolymer poly(METAC-co-HEMA) prepared as described in Example 7 was involved. A polyelectrolyte solution was prepared with the two species with a final concentration of copolymers = 32.6 wt% in 0.9 M NaCl. The molar ratio of polycation to polyanion was 2.27:1.

This solution was injected through a 1 mL syringe and a 0.028" ID microcatheter with length of 130 cm in a 8.5 cm diameter Petri dish filled with 0.9% NaCl saline solution at pH 7.1.

At similar concentrations, a formulation with 80:20 n:n poly(METAC-co-HEMA) and a poly(RO acrylamide) + polyDADMAC formulation prepared in the conditions described in Example 12 both immediately gelled when injected into saline solution. It was noticed that the solution with poly(METAC-co-HEMA) was easier to inject despite high viscosity. The gel was clearer (brighter), did not tear and was deemed less rigid and more elastic than the poly(RO acrylamide) + polyDADMAC formulation.

When compared to poly(RO acrylamide) + polyMETAC without HEMA at lower concentrations closer to that of the poly(RO acrylamide) + polyDADMAC formulation described in Example 14, it appeared that the HEMA containing PEC took a few more seconds to completely harden and had time to spread. However, it was not proved that the HEMA containing gel swelled more that the gel without HEMA.

The influence of HEMA moieties in the cationic polymer in molar proportions 10 and 20% did not significantly enhance the properties of the PEC hydrogel but appeared brighter and easier to extrude from a microcatheter.

### Example 17: Preparation of liquid embolic hydrogels for CT-scan imagery

Different PEC hydrogels were prepared and immobilized in tubes or Petri dishes filled with agar agar, which were scanned in X-ray computed tomography.

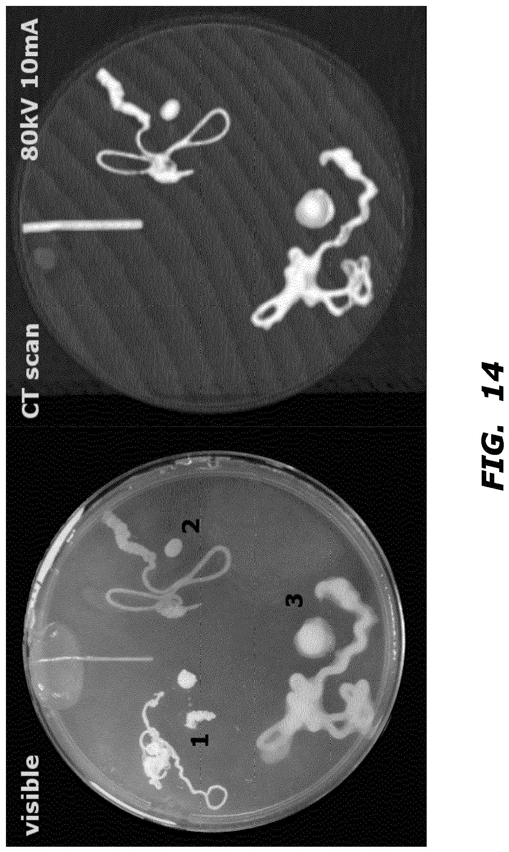

FIG. 14 shows visible light and CT-scan images comparing a gel obtained with the solution from Example 11 (#2) with another formulation (#3) and a control gel (#1) made of polyDADMAC and sodium poly(styrene sulfonate).

The formulation for gel #3 was obtained by mixing 1.2 g of the poly(RO acrylamide) obtained in Example 3 dissolved at 20.9 wt% in 2.1 M NaCl with 1.306 g of a 20.8 wt% polyDADMAC solution, resulting in a polyelectrolyte solution of 23.6% w/v total polymers and 0.9 M NaCl. The molar ratio of polycation to polyanion was 5:1.

The radiopacity of all the samples containing the poly(RO acrylamide) was demonstrated with various contrast depending on the formulation while the control sample without poly(RO acrylamide) was invisible in the scans.

### Example 18: Occlusion tests

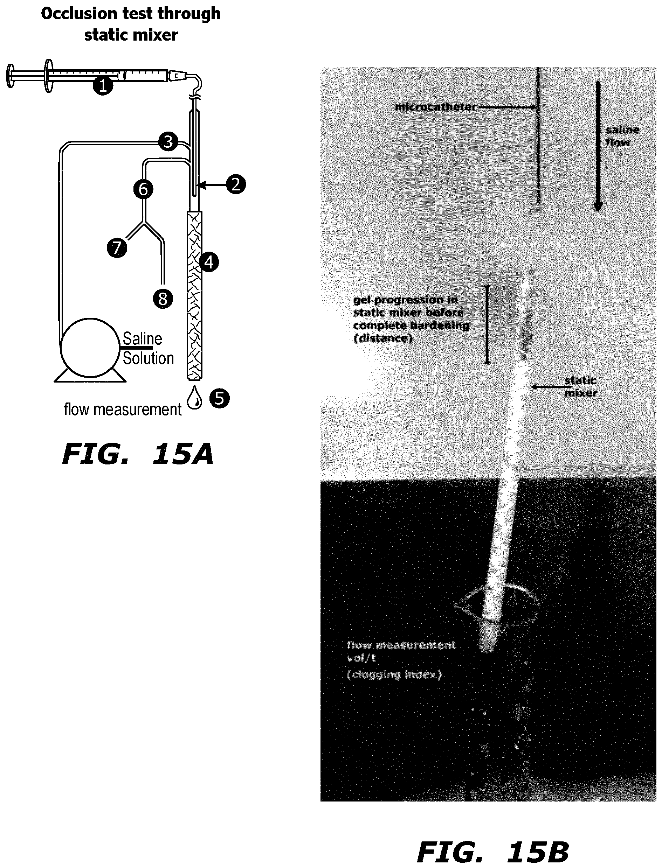

The occlusive ability of different hydrogels was evaluated *in vitro* in a saline solution flow managed with a peristaltic pump. The testing apparatus is shown in FIG. 15A, which depicts a liquid embolic injector 1, a microcatheter 2, flow of a saline solution 3, a static mixer 4, output flow measurement 5, a secondary overflow channel 6, a pressure gauge 7, and a drainage tube 8. In each test, 0.5 mL of the polyelectrolyte solution was delivered via a syringe and a microcatheter into a tube and combined with the saline solution flow upstream of a static mixer. The coacervate formed when diffused in the saline solution and carried by the stream into the static mixer. The flow rate at the exit of the static mixer was measured before each test series (50 - 60 mL/min).

Cohesive gels quickly stuck and agglomerated in the static mixer while weaker gels and slow coacervation solutions were more diffused in it or completely flushed. As the circulation was blocked, the flow was evacuated through another channel avoiding overpressure. A residual flow was always observed at the end of the static mixer because the pulsation of the pump shook the mobile part of the device and liquid could still circulate around the clog. The residual flow was measured and then the static mixer was unmounted and the length of the gel progression in the static mixer was measured.

Additionally, the pressure was measured in the secondary overflow channel providing another comparative information regarding the occlusion efficiency between different formulations. The evacuation was blocked at the time of the measurement so that the saline solution flow was completely oriented toward the occlusion site.

Each test was performed three times as sufficient volume solution available and values were averaged.

The output of the apparatus is shown in FIG. 15B with a colored gel. Each test potentially provided the following information:
- The gel progression in the static mixer gave an indication of its complete hardening time when compared to other formulations. See e.g., FIG. 15B illustrating measurement of the progression distance.
- The occlusive ability was quantified as a ratio, where 0% means no occlusion and 100% means total occlusion. The calculation is: $% clogging = \frac{\left(initial flow-flow measured after clogging\right)}{initial flow} \times 100$

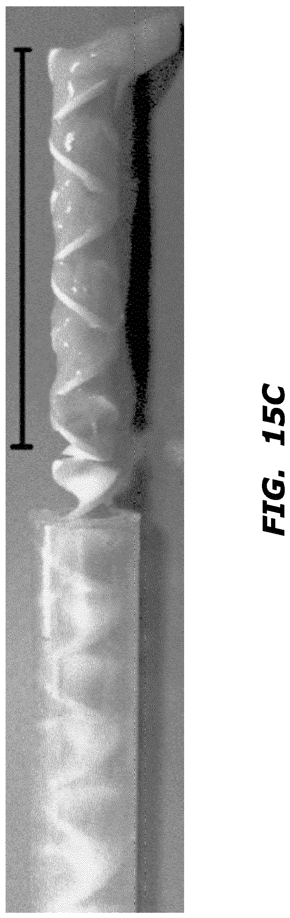

FIG. 15C shows the results of the formulation from Example 14, which obtained an "occlusion score" of 94% and an average progression length of 2.6 cm in the static mixer. Polyelectrolyte complex solutions comprising RO acrylamide polymers and copolymers with polyDADMAC and polyMETAC as polycations showed occlusion scores ranging from 50% to 95% and their progression lengths in the static mixer ranged from 2 cm up to 5 cm. Average back pressures > 0.5 bar were frequently recorded in the secondary overflow channel at the end of the procedure with similar formulations, indicating that the clot formed is able to interrupt the liquid flow.

This approach was used to compare different formulations and ascertain the influence of several parameters including:
- Solution concentration of polyelectrolytes;
- Salt concentration;
- Molar ratio of polycation to polyanion;
- Polyelectrolyte nature / copolymers; and
- Molecular weight of the polycation (polyDADMAC)

Non-radiopaque polyelectrolyte complex solutions were also evaluated with this method, for example polyDADMAC + polySSNa solutions for which occlusion scores ranged from 47% to 100% and the progression lengths in the static mixer ranged from 0.9 cm to 3.4 cm.

### Example 19: Calcium chloride trigger concentration depending on poly(RO acrylamide) batch and global polyelectrolytes concentration

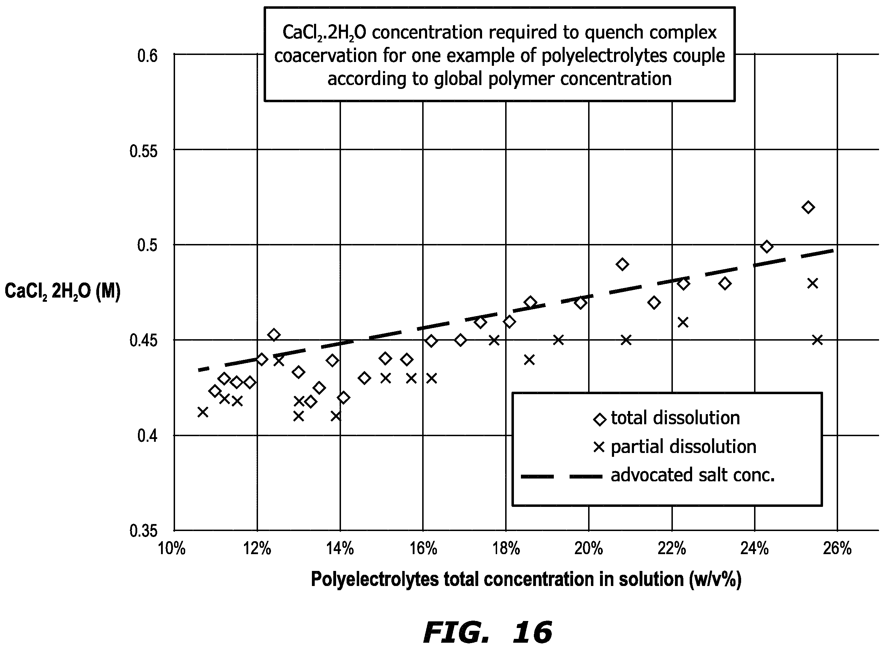

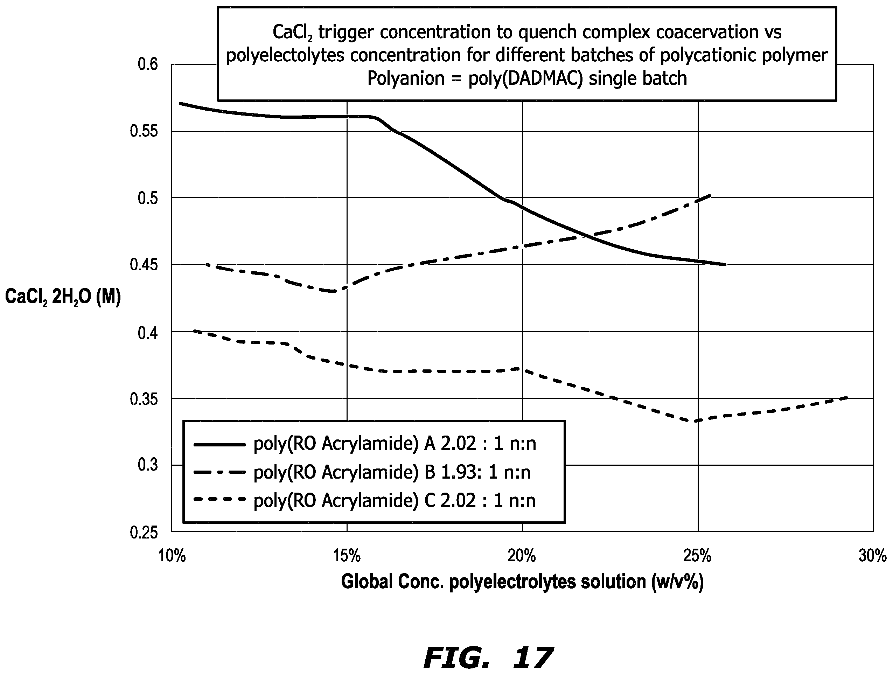

In this example, a single batch of a 39.2 wt% polyDADMAC solution with medium MW (200 - 350 kDa) was used with three different batches of poly(RO Acrylamide) solutions: A, B, C. To determine the trigger concentration, salt was slowly added in small amounts until the polyelectrolyte coacervate totally dissolved. Small adjustments were performed either by dilution or salt addition as the global polymer concentration was gradually decreased. A trend can then be visualized on a graph such as shown in FIG. 16, which shows results for 39.2 wt% polyDADMAC medium MW + 20.3 wt% poly(RO Acrylamide) [B] 1.93:1 n:n. Diamonds represent total dissolution of the coacervate, while crosses correspond to a beginning of complexation (partial dissolution). The minimum salt concentration required to quench complex coacervation according to polymers total concentration for this example only (one couple of polyelectrolytes) can be spotted on a curve (dashed line) through the diamonds and above the crosses. FIG. 17 shows the trends observed with the three different poly(RO Acrylamide) batches.

### Example 20: Calcium chloride trigger concentration depending on the polycation and global polyelectrolytes concentration

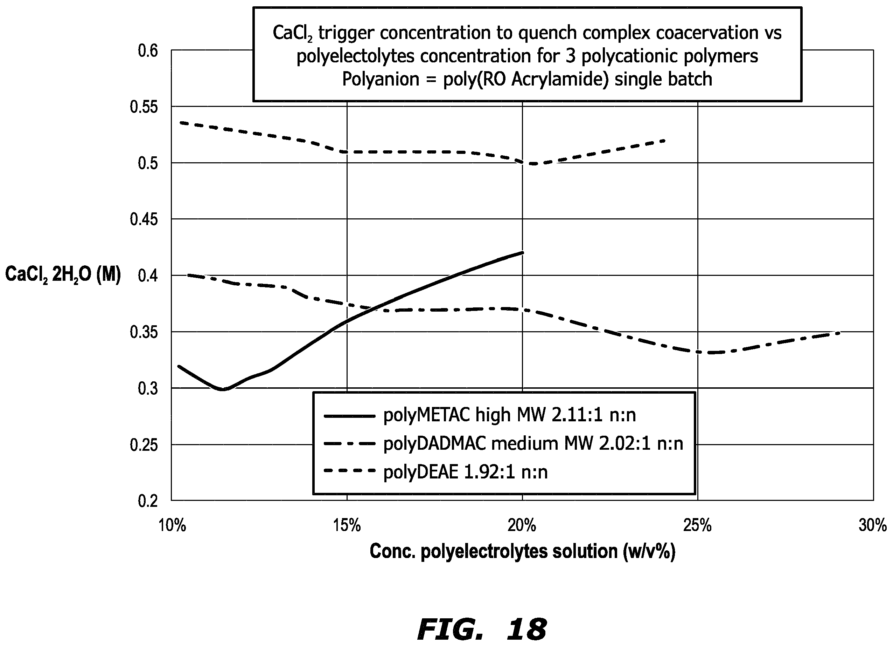

Similar to the experiment in Example 20, a single poly(RO Acrylamide) batch solution was used with various polycations in molar ratios and the CaCl₂ trigger concentration vs global polymers concentration was determined. The results shown in FIG. 18 were obtained, demonstrating the differences between the polyelectrolyte pairs.

### Example 21: Typical in vitro injection procedure through a catheter

A radiopaque embolic liquid was prepared with poly(METAC) as the polycation and poly(RO Acrylamide) as the polyanion, with a molar ratio 2.04:1 n:n using calcium chloride CaCl₂ as the doping salt. Both macromolecules were polymerized, purified and concentrated following similar procedure as described in Examples 1 to 6. The resulting solution had a global polymer concentration of 23.4% w/v, a CaCl₂ concentration of 0.66 M, and pH of 6.8.

The solution was loaded in a 3 mL syringe, and air bubbles were removed by centrifugation. The syringe was then connected to a 3-way luer stopcock having a random volume syringe filled with a 0.9% saline solution connected to the other input and a microcatheter for embolization procedures connected to the output. The 3-way stopcock and the catheter were flushed with 0.9% saline solution before liquid embolic injection. The open end of the catheter was placed into a petri dish containing 0.9% saline solution and the solution was injected into the saline solution.

This protocol allowed the collection of information including:
- injectability depending on the viscosity of the liquid embolic solution
- gelling speed in physiological medium
- cohesion of the resulting gel
- adhesion to catheter end tip

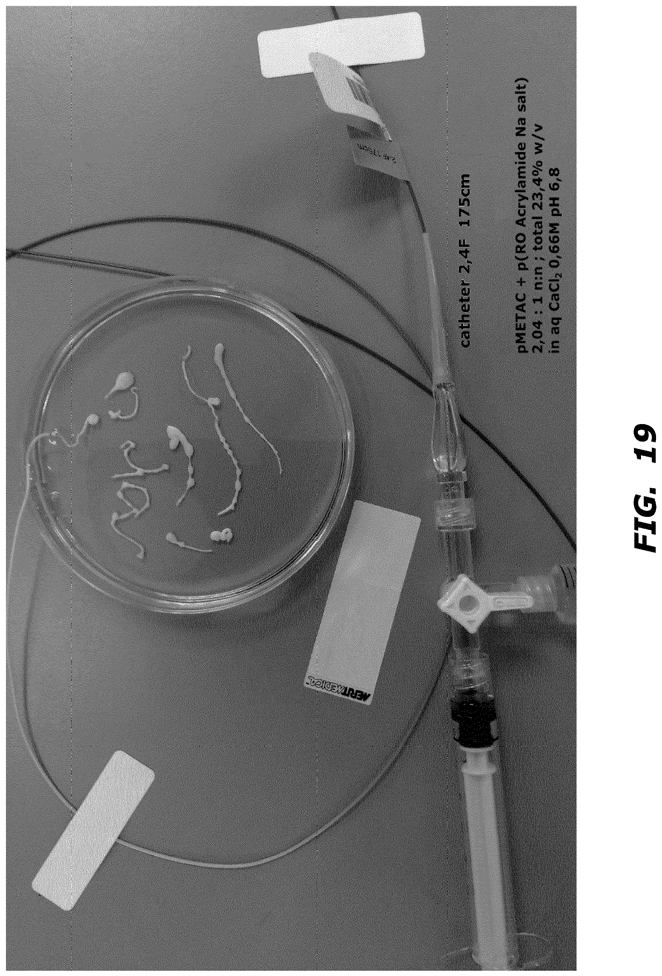

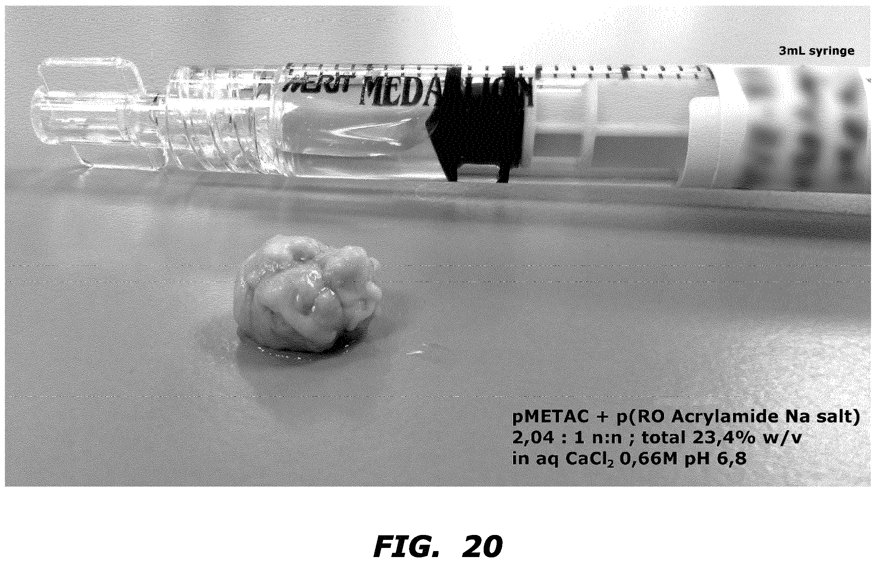

The experimental setup and a resulting gel is shown in FIG. 19. In this example, the solution was easily delivered and gellified in one second when diffused in saline. The sharpness of the filaments demonstrated a quick cohesion of the embolic solution without spreading. The filaments were removed from the petri dish and packed together. As shown in FIG. 20, the packed coacervate filaments fused into a cohesive, soft, non-flowing hydrogel.

### Example 22: Preparation of a poly(styrenesulfonate) sodium salt solution by polymerization

Poly(styrenesulfonate) sodium salt polymers were prepared starting from the monomer sodium 4-vinylbenzene sulfonic acid. The monomer was polymerized with the initiator couple APS/TEMED at pH 6 -8. The process was similar to that described in Example 1. The resulting polymer was isolated and purified by precipitation and washings / filtrations in ethanol.

If desired, the counterion (e.g. Na⁺, K⁺, Li⁺, etc.) of the sulfonate can be selected by purchasing poly(styrene sulfonic acid) or the corresponding monomer (4-styrenesulfonic acid) and neutralizing them with a particular base (NaOH, KOH, LiOH, etc.).

## Claims

1. A radiopaque embolic liquid, comprising in aqueous solution:
a polyanionic polymer;
a polycationic polymer; and
a chaotropic salt at least partially dissociated into bivalent or monovalent cations and anions, where said salt is present in a concentration greater than a trigger concentration for quenching the complexation of the polyanionic polymer and the polycationic polymer,
wherein at least one of the polyanionic polymer and the polycationic polymer comprises a radiopaque monomer.

2. The radiopaque embolic liquid of claim 1, wherein the radiopaque monomer is an iodonated monomer, preferably wherein the radiopaque monomer comprises an iodobenzene moiety.

3. The radiopaque embolic liquid of claim 1 or claim 2, wherein the polyanionic polymer comprises a radiopaque anionic monomer according to Formula I: 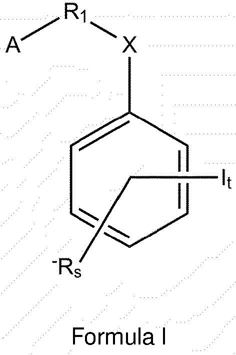 where R⁻ is an anionic substituent selected from COO⁻, SO₃⁻, PO₄³⁻, and PO₃²⁻; X is a linker selected from -C-, -NH-, and -CONH-; R₁ is a spacer comprising an alkyl, optionally substituted with one or more of -O-, -OH, and -CO; A is a polymerizable function; *s* is 1 to 3, *t* is 1 to 3, and s *+ t* is 2 to 5.

4. The radiopaque embolic liquid of claim 1 or claim 2, wherein the polyanionic polymer comprises a radiopaque anionic monomer according to Formula la: 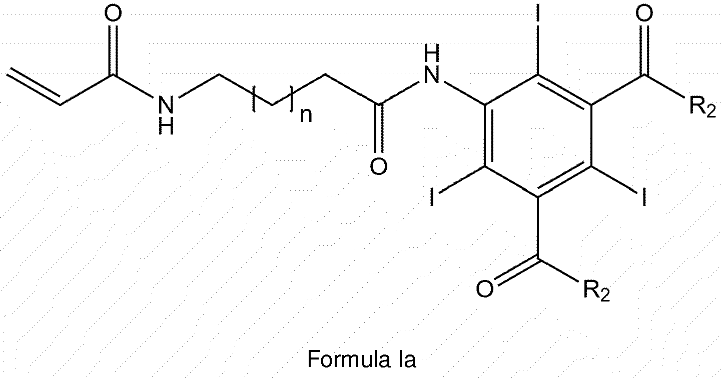
wherein n is 0-12 and wherein R₂ is either an oxygen-containing moiety or a nitrogen-containing moiety; or
wherein the polyanionic polymer comprises a radiopaque anionic monomer according to Formula Ib: 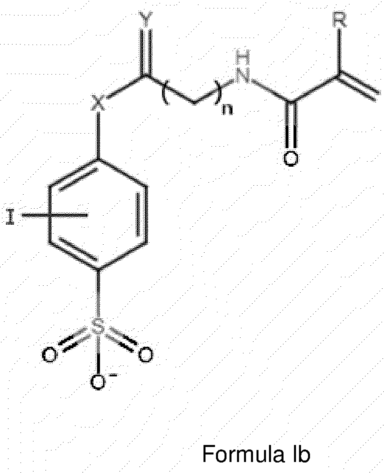
wherein R is CH₃ or H; X is O or NH; Y is O or H₂; and n is 1-4; or wherein the polyanionic polymer comprises a radiopaque anionic monomer according to Formula Ic: 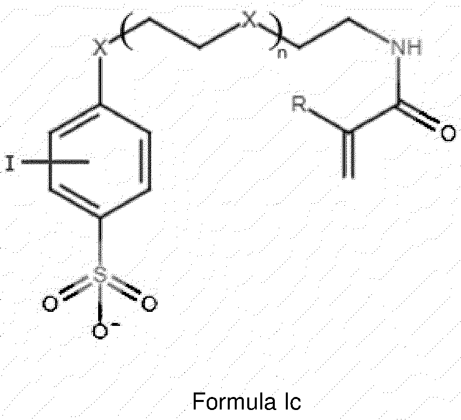
wherein R is CH₃ or H; X is O or NH; and n is 1-4.

5. The radiopaque embolic liquid of any one of claims 1 to 4, wherein the polyanionic polymer is a copolymer comprising an anionic monomer and a radiopaque monomer , preferably
wherein the polyanionic polymer is a copolymer according to Formula II: 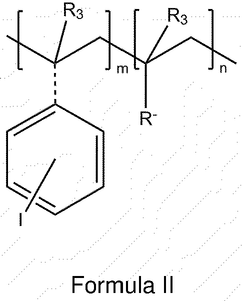
wherein R⁻ is an anionic substituent selected from COO⁻, SO₃⁻, PO₄³⁻, and PO₃²; and R₃ is H or methyl.

6. The radiopaque embolic liquid of any one of claims 1 to 5, wherein the polyanionic polymer is a copolymer comprising an anionic monomer and an uncharged monomer.

7. The radiopaque embolic liquid of any one of claims 1 to 6, wherein the polycationic polymer is a polyamine, or comprises at least one cationic monomer derived from one of pyridinium, imidazolium, guanidinium, and pyrrole.

8. The radiopaque embolic liquid of any one of claims 1 to 7, wherein the polycationic polymer is a copolymer comprising a cationic monomer and an uncharged monomer.

9. The radiopaque embolic liquid of any one of claims 1 to 8, wherein the chaotropic salt is a monovalent salt or a bivalent salt, preferably selected from NaCl, MgCl₂, and CaCl₂.

10. The radiopaque embolic liquid of any one of claims 1 to 9, wherein the chaotropic salt is present in a concentration of about 0.3 M to about 6 M.

11. The radiopaque embolic liquid of any one of claims 1 to 10, having a pH of about 6 to about 8.

12. The radiopaque embolic liquid of any one of claims 1 to 11, wherein the total concentration of polyanionic polymer and polycationic polymer in the polyelectrolyte solution is about 10% w/v to about 30% w/v and/or wherein the molar ratio of polycationic polymer to polyanionic polymer is about 1.05:1 to about 10:1.

13. The radiopaque embolic liquid of any one of claims 1 to 12, wherein the radiopaque embolic liquid is configured to undergo coacervation and precipitate a hydrogel when disposed in a medium of lower osmolality.

14. A method of making the radiopaque embolic liquid of any one of claims 1-13, comprising:
combining in aqueous solution an amount of a polyanionic polymer and an amount of a polycationic polymer with an amount of a chaotropic salt to form a polyelectrolyte solution,
wherein the amount of chaotropic salt provides a salt concentration greater than a trigger concentration for quenching the complexation of the polyanionic polymer and the polycationic polymer, and
wherein at least one of the polyanionic polymer and the polycationic polymer comprises a radiopaque monomer.

15. The method of claim 14, wherein the combining step comprises:
providing an aqueous solution comprising the amount of chaotropic salt and the amount of one of the polyanionic polymer and the polycationic polymer;
adding the amount of the other one of the polyanionic polymer and the polycationic polymer to the aqueous solution.

## Patentansprüche

1. Strahlenundurchlässiges flüssiges Embolisat, das in einer wässrigen Lösung Folgendes umfasst:
ein polyanionisches Polymer;
ein polykationisches Polymer; und
ein chaotropes Salz, das zumindest teilweise in zweiwertige oder einwertige Kationen und Anionen dissoziiert ist, wobei das Salz in einer Konzentration vorliegt, die größer ist als eine auslösende Konzentration zum Quenchen der Komplexierung des polyanionischen Polymers und des polykationischen Polymers,
wobei mindestens eines des polyanionischen Polymers und des polykationischen Polymers ein strahlenundurchlässiges Monomer umfasst.

2. Strahlenundurchlässiges flüssiges Embolisat nach Anspruch 1, wobei das strahlenundurchlässige Monomer ein iodiertes Monomer ist, bevorzugt wobei das strahlenundurchlässige Monomer eine Iodbenzoleinheit umfasst.

3. Strahlenundurchlässiges flüssiges Embolisat nach Anspruch 1 oder Anspruch 2, wobei das polyanionische Polymer ein strahlenundurchlässiges anionisches Monomer gemäß der Formel I umfasst: 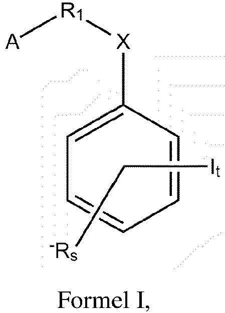 wo R⁻ ein anionischer Substituent ist, der aus COO⁻, SO₃⁻, PO₄³⁻ und PO₃²⁻ ausgewählt ist; X ein Linker ist, der aus -C-, -NH- und -CONH- ausgewählt ist; R₁ ein Spacer ist, der ein Alkyl umfasst, das optional mit einem oder mehreren von -O-, -OH und -CO substituiert ist; A eine polymerisierbare Funktion ist; *s* 1 bis 3 ist, *t* 1 bis 3 ist und *s + t* 2 bis 5 ist.

4. Strahlenundurchlässiges flüssiges Embolisat nach Anspruch 1 oder Anspruch 2, wobei das polyanionische Polymer ein strahlenundurchlässiges anionisches Monomer gemäß der Formel Ia umfasst: 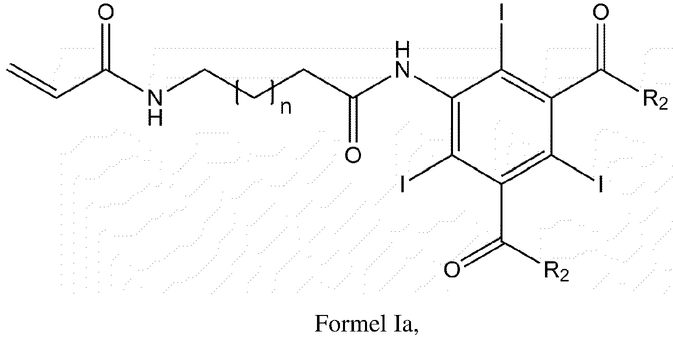
wobei n 0-12 ist und wobei R₂ entweder eine Sauerstoff-enthaltende Einheit oder eine Stickstoff-enthaltende Einheit ist; oder
wobei das polyanionische Polymer ein strahlenundurchlässiges anionisches Monomer gemäß der Formel Ib umfasst: 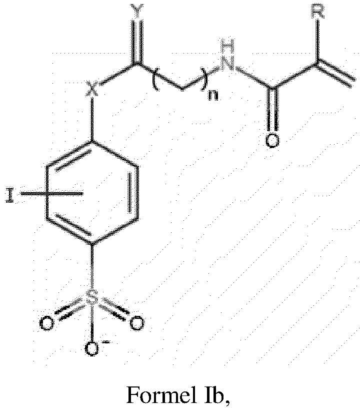
wobei R CH₃ oder H ist; X O oder NH ist; Y O oder H₂ ist; und n 1-4 ist; oder wobei das polyanionische Polymer ein strahlenundurchlässiges anionisches Monomer gemäß der Formel Ic umfasst: 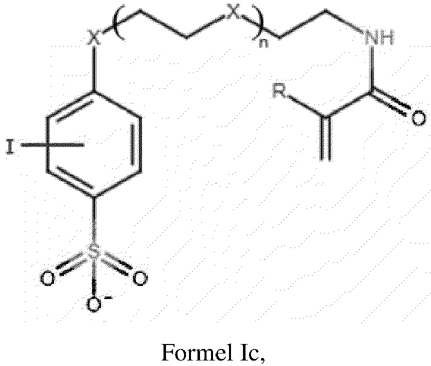
wobei R CH₃ oder H ist; X O oder NH ist; und n 1-4 ist.

5. Strahlenundurchlässiges flüssiges Embolisat nach einem der Ansprüche 1 bis 4, wobei das polyanionische Polymer ein Copolymer ist, das ein anionisches Monomer und ein strahlenundurchlässiges Monomer umfasst, bevorzugt
wobei das polyanionische Polymer ein Copolymer gemäß der Formel II ist: 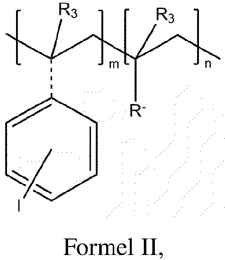
wobei R⁻ ein anionischer Substituent ist, der aus COO⁻, SO₃⁻, PO₄³⁻ und PO₃²⁻ ausgewählt ist; und R₃ H oder Methyl ist.

6. Strahlenundurchlässiges flüssiges Embolisat nach einem der Ansprüche 1 bis 5, wobei das polyanionische Polymer ein Copolymer ist, das ein anionisches Monomer und ein ungeladenes Monomer umfasst.

7. Strahlenundurchlässiges flüssiges Embolisat nach einem der Ansprüche 1 bis 6, wobei das polykationische Polymer ein Polyamin ist oder mindestens ein kationisches Monomer umfasst, das von einem von Pyridinium, Imidazolium, Guanidinium und Pyrrol abgeleitet ist.

8. Strahlenundurchlässiges flüssiges Embolisat nach einem der Ansprüche 1 bis 7, wobei das polykationische Polymer ein Copolymer ist, das ein kationisches Monomer und ein ungeladenes Monomer umfasst.

9. Strahlenundurchlässiges flüssiges Embolisat nach einem der Ansprüche 1 bis 8, wobei das chaotrope Salz ein einwertiges Salz oder ein zweiwertiges Salz ist, das bevorzugt aus NaCl, MgCl₂ und CaCl₂ ausgewählt ist.

10. Strahlenundurchlässiges flüssiges Embolisat nach einem der Ansprüche 1 bis 9, wobei das chaotrope Salz in einer Konzentration von etwa 0,3 M bis etwa 6 M vorliegt.

11. Strahlenundurchlässiges flüssiges Embolisat nach einem der Ansprüche 1 bis 10, das einen pH von etwa 6 bis etwa 8 aufweist.

12. Strahlenundurchlässiges flüssiges Embolisat nach einem der Ansprüche 1 bis 11, wobei die gesamte Konzentration von polyanionischem Polymer und polykationischem Polymer in der Polyelektrolytlösung etwa 10 % (m/V) bis etwa 30 % (m/V) beträgt und/oder wobei das Molverhältnis von polykationischem Polymer zu polyanionischem Polymer etwa 1,05 : 1 bis etwa 10 : 1 beträgt.

13. Strahlenundurchlässiges flüssiges Embolisat nach einem der Ansprüche 1 bis 12, wobei das strahlenundurchlässige flüssige Embolisat konfiguriert ist, um, bei Abgeben in ein Medium von geringerer Osmolalität, Koazervation zu durchlaufen und ein Hydrogel zu präzipitieren.

14. Verfahren zur Herstellung des strahlenundurchlässigen flüssigen Embolisats nach einem der Ansprüche 1-13, das Folgendes umfasst:
Vereinigen in einer wässrigen Lösung einer Menge eines polyanionischen Polymers und einer Menge eines polykationischen Polymers mit einer Menge eines chaotropen Salzes, um eine Polyelektrolytlösung zu bilden,
wobei die Menge des chaotropen Salzes eine Salzkonzentration bereitstellt, die größer ist als eine auslösende Konzentration zum Quenchen der Komplexierung des polyanionischen Polymers und des polykationischen Polymers und
wobei mindestens eines des polyanionischen Polymers und des polykationischen Polymers ein strahlenundurchlässiges Monomer umfasst.

15. Verfahren nach Anspruch 14, wobei der Vereinigungsschritt Folgendes umfasst:
Bereitstellen einer wässrigen Lösung, die die Menge des chaotropen Salzes und die Menge eines des polyanionischen Polymers und des polykationischen Polymers umfasst;
Zugeben der Menge des anderen des polyanionischen Polymers und des polykationischen Polymers zu der wässrigen Lösung.

## Revendications

1. Liquide embolique radio-opaque, comprenant, en solution aqueuse :
un polymère polyanionique ;
un polymère polycationique ; et
un sel chaotropique au moins partiellement dissocié en cations et en anions bivalents ou monovalents, ledit sel étant présent selon une concentration supérieure à une concentration de déclenchement permettant de stopper la complexation du polymère polyanionique et du polymère polycationique,
dans lequel au moins l'un parmi le polymère polyanionique et le polymère polycationique comprend un monomère radio-opaque.

2. Liquide embolique radio-opaque selon la revendication 1, dans lequel le monomère radio-opaque est un monomère iodé, de préférence dans lequel le monomère radio-opaque comprend un motif iodobenzène.

3. Liquide embolique radio-opaque selon la revendication 1 ou la revendication 2, dans lequel le polymère polyanionique comprend un monomère anionique radio-opaque selon la Formule I : 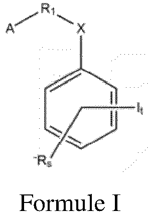 dans laquelle R⁻ est un substituant anionique choisi parmi COO⁻, SO₃⁻, PO₄³⁻ et PO₃²⁻ ; X est un bras de liaison choisi parmi -C-, -NH- et -CONH- ; R₁ est un espaceur comprenant un groupement alkyle, éventuellement substitué par un ou plusieurs parmi -O-, -OH et -CO ; A est une fonction polymérisable ; *s* va de 1 et 3, *t* va de 1 et 3, et *s + t* va de 2 et 5.

4. Liquide embolique radio-opaque selon la revendication 1 ou la revendication 2, dans lequel le polymère polyanionique comprend un monomère anionique radio-opaque selon la Formule Ia : 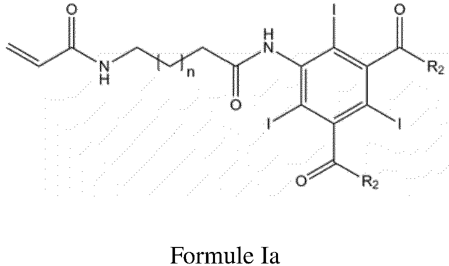
dans laquelle n vaut 0-12 et où R₂ est soit un motif contenant de l'oxygène, soit un motif contenant de l'azote ; ou
dans lequel le polymère polyanionique comprend un monomère anionique radio-opaque selon la Formule Ib : 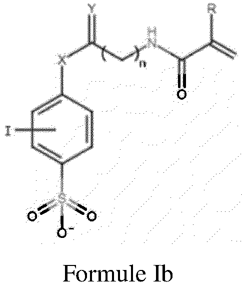
dans laquelle R est CH₃ ou H ; X est O ou NH ; Y est O ou H₂ ; et n vaut 1-4 ; ou
dans lequel le polymère polyanionique comprend un monomère anionique radio-opaque selon la Formule Ic : 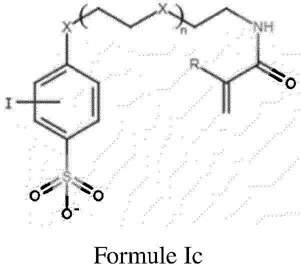
dans laquelle R est CH₃ ou H ; X est O ou NH ; et n vaut 1-4.

5. Liquide embolique radio-opaque selon l'une quelconque des revendications 1 à 4, dans lequel le polymère polyanionique est un copolymère comprenant un monomère anionique et un monomère radio-opaque, préférablement
dans lequel le polymère polyanionique est un copolymère selon la Formule II : 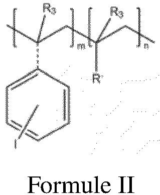 dans laquelle R⁻ est un substituant anionique choisi parmi COO⁻, SO₃⁻, PO₄³⁻ et PO₃²⁻ ; et R₃ est H ou un méthyle.

6. Liquide embolique radio-opaque selon l'une quelconque des revendications 1 à 5, dans lequel le polymère polyanionique est un copolymère comprenant un monomère anionique et un monomère non chargé.

7. Liquide embolique radio-opaque selon l'une quelconque des revendications 1 à 6, dans lequel le polymère polycationique est une polyamine, ou comprend au moins un monomère cationique dérivé de l'un parmi pyridinium, imidazolium, guanidinium et pyrrole.

8. Liquide embolique radio-opaque selon l'une quelconque des revendications 1 à 7, dans lequel le polymère polycationique est un copolymère comprenant un monomère cationique et un monomère non chargé.

9. Liquide embolique radio-opaque selon l'une quelconque des revendications 1 à 8, dans lequel le sel chaotropique est un sel monovalent ou un sel bivalent, de préférence choisi parmi NaCl, MgCl₂ et CaCl₂.

10. Liquide embolique radio-opaque selon l'une quelconque des revendications 1 à 9, dans lequel le sel chaotropique est présent selon une concentration allant d'environ 0,3 M à environ 6 M.

11. Liquide embolique radio-opaque selon l'une quelconque des revendications 1 à 10, ayant un pH allant d'environ 6 à environ 8.

12. Liquide embolique radio-opaque selon l'une quelconque des revendications 1 à 11, dans lequel la concentration totale en polymère polyanionique et en polymère polycationique dans la solution de polyélectrolytes va d'environ 10% p/v à environ 30% p/v et/ou dans lequel le rapport molaire du polymère polycationique au polymère polyanionique va d'environ 1,05:1 à environ 10:1.

13. Liquide embolique radio-opaque selon l'une quelconque des revendications 1 à 12, le liquide embolique radio-opaque étant configuré pour subir une coacervation et précipiter un hydrogel lorsqu'il est placé dans un milieu à osmolalité inférieure.

14. Méthode de fabrication du liquide embolique radio-opaque selon l'une quelconque des revendications 1 à 13, comprenant :
la combinaison, en solution aqueuse, d'une quantité d'un polymère polyanionique et d'une quantité d'un polymère polycationique avec une quantité d'un sel chaotropique pour former une solution de polyélectrolytes,
où la quantité de sel chaotropique fournit une concentration en sel supérieure à une concentration de déclenchement permettant de stopper la complexation du polymère polyanionique et du polymère polycationique, et
où au moins l'un parmi le polymère polyanionique et le polymère polycationique comprend un monomère radio-opaque.

15. Méthode selon la revendication 14, dans laquelle l'étape de combinaison comprend :
la mise à disposition d'une solution aqueuse comprenant la quantité de sel chaotropique et la quantité de l'un parmi le polymère polyanionique et le polymère polycationique ;
l'addition de la quantité de l'autre parmi le polymère polyanionique et le polymère polycationique à la solution aqueuse.
